# EUROPÄISCHE PATENTSCHRIFT

(11) **EP 3 217 187 B1**
(45) Veröffentlichungstag und Bekanntmachung des Hinweises auf die Patenterteilung: **25.08.2021**
(21) Anmeldenummer: 17179936.4
(22) Anmeldetag: 06.07.2017
(51) Int. Cl.: G01R 33/48, G01R 33/483, A61B 5/00, A61B 5/06, A61B 6/12, G01R 33/56, G01R 33/28, A61B 6/00, A61B 5/055

(54) **VERFAHREN ZUR ERMITTLUNG VON ZWEIDIMENSIONALEN BILDDATEN EINER SCHNITTFLÄCHE EINES ERFASSUNGSVOLUMENS IM RAHMEN EINER MAGNETRESONANZBILDGEBUNG**
METHOD FOR DETERMINING TWO-DIMENSIONAL IMAGE DATA OF A SLICE OF AN IMAGE ACQUISITION VOLUME IN THE COURSE OF MAGNETIC RESONANCE IMAGING
PROCÉDÉ POUR DÉTERMINER DES DONNÉES D'IMAGE EN DEUX DIMENSIONS D'UNE TRANCHE D'UN VOLUME D'IMAGERIE DANS LE CADRE D'UNE IMAGERIE A RÉSONANCE MAGNÉTIQUE

(30) Priorität: 29.07.2016 DE 102016214061
(43) Veröffentlichungstag der Anmeldung: 13.09.2017
(73) Patentinhaber: Siemens Healthcare GmbH, 91052 Erlangen (DE)
(72) Erfinder: Ertel, Dirk, 91301 Forchheim (DE); Kyriakou, Yiannis, 91080 Spardorf (DE)

(56) Entgegenhaltungen:
- WO-A1-2014/044314
- DE-A1-102010 062 340
- WANG GE ET AL: "Vision 20/20: Simultaneous CT-MRI - Next chapter of multimodality imaging", MEDICAL PHYSICS, AIP, MELVILLE, NY, US, Bd. 42, Nr. 10, 17. September 2015 (2015-09-17), Seiten 5879-5889, XP012200566, ISSN: 0094-2405, DOI: 10.1118/1.4929559 [gefunden am 1901-01-01]
- FAHRIG R ET AL: "A truly hybrid interventional MR/X-ray system: feasibility demonstration.", JOURNAL OF MAGNETIC RESONANCE IMAGING : JMRI FEB 2001, Bd. 13, Nr. 2, Februar 2001 (2001-02), Seiten 294-300, XP055375632, ISSN: 1053-1807
- ELGORT D. ET AL.: "A Method For Realtime Automated Scan Plane Selection", PROCEEDINGS OF THE INTERNATIONAL SOCIETY FOR MAGNETIC RESONANCE IN MEDICINE, 10TH SCIENTIFIC MEETING AND EXHIBITION, 18. Mai 2002 (2002-05-18), XP040586977, Hawai'i, USA
- ELAYAPERUMAL SANTHI ET AL: "Autonomous Real-Time Interventional Scan Plane Control With a 3-D Shape-Sensing Needle", IEEE TRANSACTIONS ON MEDICAL IMAGING, IEEE SERVICE CENTER, PISCATAWAY, NJ, US, Bd. 33, Nr. 11, 23. Juni 2014 (2014-06-23) , Seiten 2128-2139, XP011562847, ISSN: 0278-0062, DOI: 10.1109/TMI.2014.2332354 [gefunden am 2014-10-28]
- PAN L. ET AL.: "An Integrated System for Catheter Tracking and Visualization in MR-Guided Cardiovascular Interventions", PROCEEDINGS OF THE INTERNATIONAL SOCIETY FOR MAGNETIC RESONANCE IN MEDICINE, 19TH ANNUAL MEETING & EXHIBITION, 7. Mai 2011 (2011-05-07), Seite 195, XP040618621, Montréal, Canada

## Beschreibung

Die Erfindung betrifft ein Verfahren zur Ermittlung von zweidimensionalen Bilddaten wenigstens einer Schnittfläche eines Erfassungsvolumens im Rahmen einer Magnetresonanzbildgebung. Daneben betrifft die Erfindung eine kombinierte Magnetresonanz- und Röntgenvorrichtung, ein Computerprogramm und einen elektronisch lesbaren Datenträger.

Magnetresonanzgeräte werden im medizinischen Bereich häufig zur diagnostischen Bildgebung genutzt. Daneben werden Magnetresonanzgeräte jedoch auch im Rahmen von interventionellen Prozeduren eingesetzt, um diese Prozeduren durch eine echtzeitnahe Bildgebung zu unterstützen.

Ein typischer Anwendungsfall ist die Unterstützung eines Benutzers, indem eine echtzeitnahe Magnetresonanzbildgebung mit beispielsweise wenigstens 5, 10, 20 oder 30 Bildern pro Sekunde genutzt wird, um ihm eine momentane Situation in einem relevanten Bereich, beispielsweise im Inneren eines Patienten, zu visualisieren. Dies ist besonders dann relevant, wenn medizinische Instrumente, beispielsweise ein Katheter, an eine bestimmte Position geführt werden sollen. In diesem Fall kann einem Benutzer durch eine echtzeitnahe Bildgebung eine zielgenaue Führung des medizinischen Instruments ermöglicht werden.

Um eine Bildgebung mit einer ausreichend hohen Wiederholrate zu ermöglichen, wird hierbei typischerweise eine zweidimensionale Magnetresonanzbildgebung genutzt, bei der nur eine beziehungsweise nur einzelne Schichten angeregt und ihre Messdaten erfasst und visualisiert werden. Problematisch ist hierbei, dass der relevante Bereich eines medizinischen Instruments, beispielsweise eine Katheterspitze, hierbei die momentan erfasste Schicht verlassen kann, womit die erfasste Schicht manuell nachgeführt werden muss. Eine manuelle Nachführung der erfassten Schicht durch einen Nutzer stellt eine zusätzliche Belastung für diesen dar und kann zudem die für den Eingriff erforderliche Zeit verlängern. Ein ähnliches Problem tritt selbst dann auf, wenn eine Volumenbildgebung genutzt wird, da die Visualisierung für einen Nutzer typischerweise in Form einer Schnittebene erfolgt. Auch bei einer Volumenbildgebung ist es somit erforderlich, die dargestellte Schnittebene nachzuführen.

Der Artikel Fahrig R. et al., "A truly hybrid interventional MR/ X-ray system: Feasibility Demonstration", JMRI, 294-300 (2001), beschreibt eine MR-/Röntgenstrahl-Hybrideinrichtung, in welcher Röntgeneinrichtung und Magnetresonanzvorrichtung starr gekoppelt sind. Ein Katheter wird mithilfe der Röntgenstrahleinrichtung verfolgt, während physiologische Gegebenheiten mittels der Magnetresonanzvorrichtung visualisiert werden.

Eine kombinierte Magnetresonanz- und Röntgenvorrichtung ist auch in dem Artikel Wang G. et al., "Vision 20/20: Simultaneous CT-MRI - Next chapter of multimodality imaging", Med. Phys. 42(10), 5879-5889 (2015), beschrieben.

Der Artikel Elayaperumal S. et al., "Autonomous Real-Time Interventional Scan Plane Control With a 3-D Shape-Sensing Needle", IEEE Trans. Med. Imag., vol. 33, no. 11, 2128-2139 (2014) beschreibt das Nachführung einer Bildgebungsebene in der MRT bei einem interventionellen Eingriff.

Der Erfindung liegt somit die Aufgabe zugrunde, ein Verfahren zur Magnetresonanzbildgebung anzugeben, dass eine demgegenüber verbesserte Nachführung einer erfassten Messschicht beziehungsweise einer dargestellten Schnittebene ermöglicht.

Die Aufgabe wird erfindungsgemäß durch ein Verfahren zur Ermittlung von zweidimensionalen Bilddaten wenigstens einer Schnittfläche eines Erfassungsvolumens im Rahmen einer Magnetresonanzbildgebung durch eine kombinierte Magnetresonanz-und Röntgenvorrichtung, die eine Magnetresonanzeinrichtung und eine Röntgeneinrichtung umfasst, deren zur Bildgebung genutzte Koordinatensysteme durch eine mechanische Kopplung der Röntgeneinrichtung mit der Magnetresonanzeinrichtung zueinander registriert sind, gelöst, dass die folgenden Schritte umfasst:
- Ansteuern der Röntgeneinrichtung zum Erfassen wenigstens eines Röntgenbilds, das ein Objekt, insbesondere ein medizinisches Instrument, zumindest teilweise abbildet,
- Ermitteln wenigstens einer Objektinformation, die eine Position und/oder eine Ausrichtung und/oder wenigstens ein Merkmal des Objekts betrifft, durch eine Bildverarbeitung des Röntgenbilds,
- Ermitteln wenigstens eines Schnittflächenparameters, der eine Anordnung der Schnittfläche in dem Erfassungsvolumen vorgibt, in Abhängigkeit der Objektinformation,
- Ansteuern der Magnetresonanzeinrichtung zur Erfassung von die Schnittfläche betreffenden Messdaten, und
- Berechnen der zweidimensionalen Bilddaten aus den Messdaten,
wobei das Ansteuern der Magnetresonanzeinrichtung und/oder die Berechnung der zweidimensionalen Bilddaten in Abhängigkeit des Schnittflächenparameters erfolgt.

Erfindungsgemäß wird vorgeschlagen auszunutzen, dass in einigen Einrichtungen sowohl eine Röntgen- als auch eine Magnetresonanzbildgebung möglich ist, wobei die zur Bildgebung genutzten Koordinatensysteme eine definierte Lage zueinander aufweisen, also zueinander registriert sind. Dies kann beispielsweise bei sogenannten Angio-MR-Scannern der Fall sein. Es ist bekannt, dass eine Röntgenbildgebung mit hohen Bildraten bei relativ niedrigen Strahlungsdosen möglich ist, was beispielsweise im Rahmen der Fluoroskopie genutzt wird. Viele Objekte, insbesondere viele medizinische Instrumente, werden bei einer Röntgenbildgebung mit hohem Kontrast abgebildet. Bereits bei niedrigen Röntgendosen sind somit bestimmte Merkmale dieser Instrumente, beispielsweise die Position einer Katheterspitze, leicht zu erkennen. Dies wird erfindungsgemäß dazu genutzt, die zweidimensionale Magnetresonanzbildgebung automatisch nachzuführen, indem aus dem Röntgenbild die Objektinformation ermittelt wird und diese im Rahmen der Ansteuerung der Magnetresonanzeinrichtung beziehungsweise der Berechnung der Bilddaten genutzt wird, womit eine bezüglich des Objekts gewählte Schnittfläche automatisch nachgeführt werden kann.

Ein Benutzter der kombinierten Magnetresonanz- und Röntgenvorrichtung wird somit entlastet, da eine Schichtnachführung nicht mehr manuell erfolgen muss. Gegenüber einer rein magnetresonanzbasierten Schichtnachführung ermöglicht das erfindungsgemäße Verfahren ein schnelleres und robusteres Nachführen der Bildgebung. Die berechneten zweidimensionalen Bilddaten können beispielsweise auf einer Anzeigeeinrichtung dargestellt werden. Dies kann direkt erfolgen, es ist jedoch auch möglich, die zweidimensionalen Bilddaten zunächst weiter zu verarbeiten, beispielsweise um zusätzliche Informationen zu überlagern.

Für das erfindungsgemäße Verfahren ist es wesentlich, dass die zur Bildgebung genutzten Koordinatensysteme zueinander registriert sind. Die Registrierung der genutzten Koordinatensysteme kann starr sein, beispielsweise indem eine starre Kopplung der Magnetresonanzeinrichtung und der Röntgeneinrichtung vorgesehen wird. Beispielsweise können eine Röntgenröhre und ein Röntgendetektor ortsfest zu dem Haupt- und den Gradientenmagneten der Magnetresonanzeinrichtung angeordnet sein. Bei einer starren Registrierung kann auch ein gemeinsames Koordinatensystem zur Bildgebung durch beide Einrichtungen genutzt werden.

Es ist jedoch auch möglich, eine flexible, automatisch angepasste Registrierung zu nutzen. Beispielsweise können Komponenten der Röntgeneinrichtung, insbesondere die Röntgenröhre und der Röntgendetektor, derartig an den Komponenten der Magnetresonanzeinrichtung, insbesondere den Magneten, gelagert sein, dass sie gegenüber diesen verschieb- und/oder verschwenkbar sind. Das Verschieben und/oder Verschwenken kann manuell oder durch Aktoren der kombinierten Magnetresonanz- und Röntgeneinrichtung erfolgen. Es können Sensoren vorgesehen sein, die die relative Verschiebung und/oder Verschwenkung erfassen und/oder diese kann aufgrund einer entsprechenden Ansteuerung der Aktoren bekannt sein. Aufgrund der bekannten Verschiebung beziehungsweise Verschwenkung ist somit zu jedem Zeitpunkt auch die relative Lage und Orientierung der zur Bildgebung genutzten Koordinatensysteme zueinander bekannt, womit diese zueinander registriert sind.

Diese Registrierung ermöglicht es, Informationen zwischen diesen Koordinatensystemen zu übertragen. Hierbei kann es sich beispielsweise um Koordinaten eines Punktes, bestimmte Richtungen, Lagen und/oder Formen von Kurven, insbesondere Linien, und/oder Flächen, insbesondere Ebenen, und/oder Informationen über bestimmte in den Bilddaten erkannte Merkmale handeln. Unter dem Koordinatensystem der Magnetresonanzbildgebung ist in diesem Zusammenhang ein dreidimensionales Ortsraumkoordinatensystem der Magnetresonanzeinrichtung zu verstehen, das beispielsweise durch die Richtungen der Schichtselektions-, Frequenz- und/oder Phasenkodiergradienten aufgespannt werden kann oder eine definierte Lage bezüglich dieser Richtungen aufweist. Das Koordinatensystem der Röntgeneinrichtung kann als dreidimensionales Koordinatensystem definiert sein, dessen Position und Orientierung durch die Position und Orientierung des Röntgendetektors und/oder der Röntgenquelle festgelegt wird. Bei einer Koordinatenübertragung ist typischerweise zu berücksichtigen, dass eine erfasste Röntgenintensität an einem Detektorelement von dem Linienintegral des Absorptionskoeffizienten zwischen diesem Detektorelement und der Röntgenquelle abhängt. Sind keine weiteren Randbedingungen bekannt, kann eine Position eines bestimmten in dem Röntgenbild erkannten Merkmals in einem dreidimensionalen der Röntgeneinrichtung zugeordneten Koordinatensystem somit nur insoweit bestimmt werden, dass das Merkmal auf einer entsprechenden Verbindungslinie liegt.

Die Schnittfläche kann im erfindungsgemäßen Verfahren insbesondere eine Schnittebene sein, da dies eine besonders einfache Erfassung der Messdaten ermöglicht. Prinzipiell ist es jedoch auch möglich, gekrümmte Schnittflächen zu nutzen. Die Schnittfläche kann eine Schichtdicke senkrecht zu der Schnittfläche aufweisen, über die Magnetresonanzsignale des Objekts beziehungsweise weiterer Objekte im Erfassungsvolumen integriert werden. Die Schichtdicke der Schnittfläche kann der Schichtdicke einer im Rahmen der Magnetresonanzbildgebung angeregten Schicht entsprechen. Es ist jedoch auch möglich, dass in Richtung der Schichtselektion im Rahmen der Magnetresonanzbildgebung eine Frequenz- oder Phasenkodierung erfolgt, womit die Schichtdicke der erreichten Auflösung dieser Frequenz- oder Phasenkodierung entsprechen kann oder beliebig dicker gewählt sein kann. Die Schichtdicke kann fest oder in Abhängigkeit des Schnittflächenparameters vorgegeben sein. Der Schnittflächenparameter kann eine Lage, insbesondere eine Position und/oder eine Ausrichtung, aber auch eine Form eines Messbereichs bestimmen, für den Messdaten erfasst werden. Ergänzend oder alternativ kann der Schnittflächenparameter die Lage der darzustellenden Schnittfläche im Messbereich beschreiben. Dies ist insbesondere vorteilhaft, wenn Bilddaten einer gekrümmten Schnittfläche dargestellt werden sollen.

Im Rahmen einer durch eine Magnetresonanzbildgebung unterstützten Intervention im medizinischen Bereich werden sowohl sogenannte passive als auch sogenannte aktive Instrumente genutzt. Passive Instrumente sind derart ausgebildet, dass sie während einer Magnetresonanzbildgebung genutzt werden können, ohne diese zu stören, sich zu erhitzen oder Ähnliches. Bei aktiven Instrumenten ist zusätzlich eine Positionsbestimmung für diese Instrumente vorgesehen. Dies kann beispielsweise durch Funktriangulation eines an dem aktiven Instrument vorgesehenen Transceivers erfolgen. Das erfindungsgemäße Verfahren ist besonders vorteilhaft, wenn passive Instrumente genutzt werden, da diese anderweitig nur schwer zu lokalisieren sind. Es kann jedoch auch vorteilhaft in Verbindung mit aktiven Instrumenten genutzt werden, da typischerweise nur ein einziger Punkt an diesen aktiven Instrumenten lokalisiert wird. Durch das erfindungsgemäße Verfahren wird es in diesem Fall zusätzlich ermöglicht, eine Orientierung und/oder eine mögliche Formänderung, die insbesondere bei Kathetern auftreten kann, zu berücksichtigen.

Als Objektinformation kann eine jeweilige Punktposition wenigstens eines Messpunktes in dem Röntgenbild ermittelt werden, der in einer vorgegebenen Position bezüglich der zumindest teilweisen Abbildung des Objekts liegt, wonach der Schnittflächenparameter in Abhängigkeit der Punktposition oder der Punktpositionen derart ermittelt wird, dass die Schnittfläche zumindest einen Abschnitt einer jeweiligen Verbindungslinie zwischen einem dem jeweiligen Messpunkt zugeordneten Röntgendetektorelement und einer Röntgenquelle der Röntgeneinrichtung umfasst. Hierzu kann eine Erkennung des Objekts beziehungsweise eines bestimmten Punktes am Objekt durchgeführt werden. Dies kann beispielsweise durch eine Merkmalserkennung im Röntgenbild, beispielsweise durch eine Erkennung von skaleninvarianten Merkmalen, erfolgen. Es ist jedoch auch möglich, das Objekt im Röntgenbild zu segmentieren beziehungsweise mehrere Bereich im Röntgenbild zu segmentieren und zu klassifizieren, um das Objekt oder bestimmte Teile des Objekts zu erkennen und hieraus bestimmte Merkmale, beispielsweise das Ende eines Katheters, zu erkennen. Eine Segmentierung kann kontrastbasiert, beispielsweise durch Region Growing, erfolgen.

Die Position eines entsprechenden Merkmals kann direkt dem Messpunkt entsprechen oder der Messpunkt kann bezüglich dieser Position verschoben sein. Die Verschiebung kann hierbei in eine vorgegebene Richtung erfolgen oder in Abhängigkeit der Bilddaten des Röntgenbilds. Beispielsweise kann für einen Katheter oder ein anderes längliches Objekt zunächst eine Position des Endes des Katheters beziehungsweise Objekts bestimmt werden, anschließend eine Längsrichtung des Katheters beziehungsweise Objekts bestimmt werden und der Messpunkt kann um eine vorgegebene Distanz gegenüber der zuvor ermittelten Position in Längsrichtung des Katheters beziehungsweise des Objekts versetzt werden. Es ist auch möglich, mehrere Messpunkte zu wählen, wobei insbesondere zwei in Längsrichtung eines Objekts versetzte Messpunkte gewählt werden können. Da in diesem Fall mehrere Verbindungslinien in der Schnittfläche liegen sollen, kann im Falle einer Nutzung einer Schnittebene als Schnittfläche diese bereits vollständig durch die Auswahl dieser beiden Messpunkte vorgegeben sein.

Als Objektinformation kann wenigstens ein Richtungsvektor in dem Röntgenbild ermittelt werden, der eine bezüglich der zumindest teilweisen Abbildung des Objekts vorgegebene Richtung beschreibt, wonach der Schnittflächenparameter in Abhängigkeit des Richtungsvektors oder der Richtungsvektoren derart ermittelt wird, dass zumindest ein jeweiliger Abschnitt der Schnittfläche in dem Erfassungsraum parallel zu dem jeweiligen Richtungsvektor oder in einem vorgegebenen Winkel zu diesem steht.

Statt einen Richtungsvektor zu bestimmen, können auch zwei oder mehr Messpunkte entlang dieses Richtungsvektors bestimmt werden und es kann wie vorangehend erläutert die Schnittfläche so gewählt werden, dass sie die Verbindungslinie der zugeordneten Röntgendetektorelemente und der Röntgenquelle umfasst. Wird eine Schnittebene als Schnittfläche, gewählt, liegt diese in diesem Fall parallel zum Richtungsvektor.

Der Richtungsvektor kann bei einer gekrümmten Schnittfläche eine Tangente zu dieser Schnittfläche bilden, wobei er die Schnittfläche insbesondere an mehreren, vorzugsweise benachbarten, Punkten berührt.

Als Richtungsvektor kann vorzugsweise ein Richtungsvektor gewählt werden, der einer in dem Röntgenbild ermittelten Längsrichtung eines länglichen Objekts, insbesondere eines Katheters, entspricht. In diesem Fall resultiert eine Schnittfläche, die zumindest abschnittsweise parallel oder gewinkelt, vorzugsweise senkrecht, zu dieser Längsrichtung steht.

Das Objekt kann ein Katheter sein, wobei die Position einer Katheterspitze in dem Röntgenbild ermittelt wird und der wenigstens eine Messpunkt an der Position der Katheterspitze oder an einer bezüglich der Position der Katheterspitze vorgegebenen weiteren Position liegt, und/oder wobei der Richtungsvektor in eine Längsrichtung des Katheters gerichtet ist, die aus dem Röntgenbild ermittelt wird. Unter einer Katheterspitze ist das Ende des Katheters in seiner Längsrichtung zu verstehen. Die ermittelte Längsrichtung kann eine lokale Längsrichtung an einer bestimmten Position beziehungsweise in einem bestimmten Abschnitt des Katheters sein, beispielsweise wenn der Katheter in dem Röntgenbild gekrümmt ist. Die Längsrichtung kann insbesondere im Bereich der Katheterspitze ermittelt werden. Als Schnittflächenparameter können beispielsweise eine Position der Katheterspitze und eine Vorzugsrichtung, das heißt beispielsweise eine Längsrichtung des Katheters im Bereich der Katheterspitze, verwendet werden. Alternativ ist es beispielsweise möglich, eine gekrümmte Schnittfläche zu nutzen, wobei der Schnittflächenparameter diese derart parametrisiert, dass sie im Wesentlichen dem Verlauf des Katheters in dem Röntgenbild folgt.

Eine Gruppe der Schritte, die zumindest die Ermittlung des jeweiligen Schnittflächenparameters und die Ansteuerung der Magnetresonanzeinrichtung zur Erfassung der die jeweilige Schnittfläche betreffenden Messdaten umfasst, kann zeitlich aufeinanderfolgend wiederholt durchgeführt werden, wobei in wenigstens einer der Wiederholungen der jeweilige Schnittflächenparameter in Abhängigkeit von den in einer jeweiligen früheren Wiederholung erfassten Messdaten ermittelt wird. Hierbei ist es möglich, dass auch die weiteren Schritte des Verfahrens wiederholt werden, so dass innerhalb jeder Wiederholung oder in einem Teil der Wiederholungen jeweils zusätzlich Röntgenbilder erfasst werden und aus diesen wenigstens eine Objektinformation ermittelt wird. Hierüber kann eine Schichtnachführung implementiert werden, bei der zur Schichtnachführung sowohl wenigstens eine Objektinformation aus einem aktuell erfassten Röntgenbild als auch die Messdaten einer vorangehenden Magnetresonanzmessung berücksichtigt werden.

Alternativ ist es möglich, dass die Gruppe mehrfach nach jedem Erfassen einer Röntgenaufnahme und Ermitteln einer Objektinformation wiederholt wird. Dies kann beispielsweise vorteilhaft sein, um eine in das Erfassungsvolumen eingestrahlte Röntgendosis und somit beispielsweise eine Strahlenbelastung eines Patienten weiter zu reduzieren. Der Schnittflächenparameter für eine erste Schnittfläche kann ausschließlich in Abhängigkeit der Objektinformation ermittelt werden und eine weitere Nachführung kann die vorangehend erfassten Messdaten berücksichtigen. In vorgegebenen Abständen kann erneut ein Röntgenbild aufgenommen und eine Objektinformation ermittelt werden, um die Nachführung mit weiteren Informationen anzureichern. Die Wiederholung der Gruppe kann jedoch auch, wie später noch genauer erläutert werden wird, genutzt werden, um eine Schnittfläche, für die zweidimensionale Bilddaten erfasst und/oder berechnet und insbesondere dargestellt werden, iterativ zu bestimmen. Es kann beispielsweise durch eine wiederholte Erfassung von Messdaten und eine folgende Anpassung des Schnittflächenparameters in Abhängigkeit dieser Messdaten ein optimaler Schnittflächenparameter beziehungsweise eine optimale Schicht gesucht werden, wobei ein Anfangspunkt für diese Suche, also ein initialer Schnittflächenparameter, in Abhängigkeit der Objektinformation und somit des Röntgenbilds bestimmt werden kann. Der initiale Schnittflächenparameter kann beispielsweise wie vorangehend erläutert so bestimmt werden, dass die Schnittfläche eine Katheterspitze umfasst beziehungsweise bezüglich dieser positioniert und/oder orientiert wird.

Durch die in den früheren Wiederholungen erfassten Messdaten kann das Objekt zumindest teilweise abgebildet werden, wobei durch eine Verarbeitung dieser Messdaten eine weitere Objektinformation bestimmt wird, die die Position und/oder die Ausrichtung und/oder das oder wenigstens ein weiteres Merkmal des Objekts betrifft, wobei der Schnittflächenparameter in Abhängigkeit der weiteren Objektinformation ermittelt wird. Die weitere Objektinformation kann einen Schnittpunkt des Objekts mit der Schnittfläche beschreiben. Um diesen Schnittpunkt zu ermitteln, können zweidimensionale Bilddaten aus den Messdaten ermittelt werden, beispielsweise durch eine Fourier-Transformation, und diese zweidimensionalen Bilddaten können segmentiert beziehungsweise es kann eine Merkmalserkennung in ihnen durchgeführt werden, um den Schnittpunkt zu erkennen. Der Schnittflächenparameter kann derart gewählt werden, dass die Schnittfläche, deren Messdaten nachfolgend aufgenommen werden, den Schnittpunkt umfasst und/oder parallel oder in einem vorgegebenen Winkel, insbesondere senkrecht, zu einer Verbindungslinie zwischen dem Schnittpunkt und einem weiteren definierten Punkt, beispielsweise einer in den Röntgendaten detektierten Katheterspitze, liegt.

Während wenigstens einer der Wiederholungen kann vor dem Ermitteln des Schnittflächenparameters die Magnetresonanzeinrichtung zur Erfassung von einer Hilfsschnittfläche betreffenden Hilfsmessdaten angesteuert werden, wobei die Hilfsschnittfläche von jener Schnittfläche, deren Messdaten in der früheren Wiederholung erfasst werden, beabstandet ist und/oder in einem vorgegebenen Winkel zu dieser steht, wonach ein Hilfsschnittpunkt des Objekts mit der Hilfsschnittfläche bestimmt wird, wonach der Schnittflächenparameter derart ermittelt wird, dass die Schnittfläche den Schnittpunkt und den Hilfsschnittpunkt umfasst oder einen vorgegebenen Winkel, insbesondere 90°, mit einer Verbindungslinie zwischen dem Schnittpunkt und dem Hilfsschnittpunkt einschließt. Die Hilfsschnittfläche kann in Abhängigkeit des Röntgenbilds und/oder des Objektparameters gewählt werden. Durch das beschriebene Vorgehen kann insbesondere die Lage eines linearen Objekts, beispielsweise eines nicht zu stark gekrümmten Katheters, sehr exakt erfasst werden, womit eine Positionierung und Orientierung der Schnittfläche bezüglich dieses Objekts sehr genau möglich ist.

Die oder eine weitere Gruppe von Schritten, die zumindest die Ermittlung des jeweiligen Schnittflächenparameters und die Ansteuerung der Magnetresonanzeinrichtung zur Erfassung der jeweiligen die jeweilige Schnittfläche betreffenden Messdaten umfasst, kann wiederholt werden, wenn nach dem Erfassen der Messdaten ermittelt wird, dass die Messdaten das Objekt nicht abbilden, wobei der Schnittflächenparameter gemäß einem fest oder in Abhängigkeit der Objektinformation vorgegebenen Muster variiert wird. Es kann somit durch eine Variation des Schnittflächenparameters nach einer Schnittfläche gesucht werden, die das Objekt schneidet oder umfasst. Hierbei kann bei Nutzung einer Schnittebene als Schnittfläche die Position der Schnittfläche, insbesondere senkrecht zur Schnittfläche, und/oder die Orientierung der Schnittfläche im Erfassungsraum variiert werden. Die Objektinformation kann beispielsweise in der Form berücksichtigt werden, dass eine Längsrichtung des Objekts aus den Röntgendaten ermittelt wird und die Verschiebungen in diese Längsrichtung erfolgt und/oder eine Orientierung der Schnittfläche im Rahmen der Variation derart angepasst wird, dass sie gewinkelt, insbesondere senkrecht, zu dieser Richtung steht.

Wenn die Messdaten das Objekt zumindest teilweise abbilden, kann durch eine Verarbeitung der Messdaten eine Abbildungsinformation ermittelt werden, die beschreibt, welcher Anteil des Objekts durch die Messdaten abgebildet wird, wonach bei Erfüllung einer die Abbildungsinformation auswertenden Wiederholungsbedingung die oder eine Gruppe der Schritte, die zumindest die Ermittlung des jeweiligen Schnittflächenparameters und die Ansteuerung der Magnetresonanzeinrichtung zur Erfassung der jeweiligen die jeweilige Schnittfläche betreffenden Messdaten umfasst, wiederholt werden, wobei der Schnittflächenparameter gemäß einem fest oder in Abhängigkeit von der Objektinformation und/oder den Messdaten vorgegebenen Muster variiert wird, und bei Nichterfüllung der Wiederholungsbedingung die zweidimensionalen Bilddaten in Abhängigkeit der vorangehend erfassten Messdaten berechnet werden.

Der Schnittflächenparameter kann insbesondere in Abhängigkeit der Abbildungsinformation variiert werden, um beispielsweise ein Optimierungsverfahren durchzuführen, durch das der durch die Messdaten abgebildete Anteil des Objekts maximiert wird. Hierzu kann beispielsweise ein Gradientenverfahren durchgeführt werden, bei dem der Schnittflächenparameter variiert wird, beispielsweise um eine Orientierung und Position einer Schnittebene zu variieren, und für eine Variation in zwei mögliche Änderungsrichtungen pro Freiheitsgrad jeweils ermittelt wird, in welche Richtung sich der durch die Messdaten abgebildete Anteil des Objekts maximal erhöht. Die Abbildungsinformation kann ein numerischer Wert sein, womit sie beispielsweise direkt in einem Gradientenverfahren oder einem anderen Optimierungsverfahren ausgewertet werden kann. Die Abbildungsinformation kann beispielsweise beschreiben, wie groß die Fläche, die den Anteil des Objekts abbildet, in aus den Messdaten berechneten zweidimensionalen Bilddaten ist. Alternativ können Segmente des Objekts vorgegeben werden und es kann bestimmt werden, wie viele dieser Segmente durch die Messdaten abgebildet werden. Dies kann beispielsweise dadurch erfolgen, dass Merkmale für die einzelnen Segmente vorgegeben werden und überprüft wird, ob diese jeweils durch die Messdaten abgebildet werden. Die Vorgabe der Segmentierung kann durch Vorwissen erfolgen, beziehungsweise indem eine Datenbank genutzt wird, die Daten des Objekts speichert. Alternativ kann eine Segmentierung des Objekts auch in dem Röntgenbild erfolgen.

Die Abbildungsinformation kann direkt in einem k-Raum, also einem Raum von Ortsfrequenzen, in dem Magnetresonanzmessdaten typischer Weise erfasst werden, ermittelt werden. Dies ist in Fällen möglich, in denen einzelnen Segmenten des Objekts eindeutige, im k-Raum identifizierbare Merkmale zugeordnet werden können. Alternativ oder ergänzend können aus den Messdaten temporäre zweidimensionale Bilddaten berechnet werden und die Abbildungsinformation kann in Abhängigkeit dieser Bilddaten bestimmt werden. Bei Nichterfüllung der Wiederholungsbedingung können in diesem Fall direkt die temporären zweidimensionalen Bilddaten als ausgegebene zweidimensionale Bilddaten bereitgestellt werden.

Die Abbildungsinformation kann alternativ oder ergänzend mit Hilfe eines Verfahrens des Maschinenlernens ermittelt werden. Beispielsweise können eine Vielzahl von Lehrdaten bereitgestellt werden, die dem Format der Messdaten entsprechen können, und die unterschiedliche Anteile eines bestimmten Objekts beziehungsweise eines Objekts eines bestimmten Typs zeigen. Für diese Lehrdaten kann manuell oder durch ein beliebiges anderes Verfahren eine jeweilige Abbildungsinformation vorgegeben werden und die derart annotierten Lehrdaten können anschließend zum Trainieren eines Algorithmus des Maschinenlernens eingesetzt werden.

Die Abbildungsinformation kann in Abhängigkeit wenigstens eines Merkmals des Objekts ermittelt werden, das durch die Objektinformation und/oder ein Vorwissen über das Objekt beschrieben oder daraus ermittelt wird. Das Merkmal kann insbesondere eine Form des Objekts beziehungsweise eines oder mehrerer Abschnitte des Objekts sein. Es kann jedoch auch eine Schablone für eine Template-basierte Objekterkennung, ein skaleninvariantes Merkmal oder Ähnliches sein. Das entsprechende Merkmal kann durch eine Segmentierung des Röntgenbildes bestimmt werden. Hierbei kann das Röntgenbild, insbesondere durch eine starre oder nicht starre Registrierung, zu einem als Vorwissen bereitgestellten, das Objekt beschreibenden Datensatz, beispielsweise einem 3D-Modell des Objekts, registriert werden. Dies hat den Vorteil, dass einige Merkmale, beispielsweise eine Grundform des Objekts beziehungsweise von starren Bereichen des Objekts, exakt durch das Vorwissen bereitgestellt werden, wobei zusätzliche Informationen, die eine Verformung, eine Position und/oder eine Orientierung des Objekts betreffen, aus dem Röntgenbild ermittelt werden können.

Als Abbildungsinformation kann ein Maß dafür bestimmt werden, welcher Anteil des Objekts durch die Messdaten abgebildet wird, wobei die Wiederholungsbedingung die Abbildungsinformation mit einem vorgegebenen Grenzwert vergleicht oder ein Konvergenzkriterium auswertet, das mehrere in aufeinanderfolgenden Wiederholungen der Gruppe der Schritte ermittelte Abbildungsinformationen berücksichtigt. Beispielsweise kann vorgegeben werden, dass wenigstens 30, 50 oder 70 % des Objekts abgebildet werden. Die Auswertung eines Konvergenzkriteriums ist dann vorteilhaft, wenn ein Optimierungsverfahren genutzt werden soll, um den abgebildeten Anteil des Objekts zu maximieren. Ein solches Optimierungsverfahren beziehungsweise iteratives Verfahren kann beispielsweise das bereits erwähnte Gradientenverfahren sein.

In einigen Fällen soll das Objekt zu einem definierten Zielpunkt in dem Erfassungsvolumen geführt werden. Daher kann ein Zielpunkt in dem Erfassungsvolumen vorgegeben werden, wobei der Schnittflächenparameter derart ermittelt wird, dass die Schnittfläche den Zielpunkt umfasst. Ein entsprechender Zielpunkt kann manuell vorgegeben werden, beispielsweise in zuvor in der gleichen Messgeometrie aufgenommenen dreidimensionalen Magnetresonanzdaten. Der Zielpunkt kann jedoch auch in dem Röntgenbild vorgegeben werden. Beispielsweise kann der Schnittflächenparameter derart ermittelt werden, dass eine Schnittfläche eine Katheterspitze eines zu führenden Katheters sowie einem Zielpunkt, zu dem der Katheter geführt werden soll, umfassen.

Die Schnittfläche kann eine Schnittebene sein, wobei die Schnittfläche parallel zu einem Zentralstrahl einer Röntgenquelle der Röntgeneinrichtung liegt. Bei einer näherungsweise parallelen Röntgenstrahlung steht die Schnittfläche in diesem Fall senkrecht zur Bildebene des Röntgenbildes und wird in diesem als Linie abgebildet. Bei einer fächer- oder konusförmig abstrahlenden Röntgenquelle kann jener Strahl als Zentralstrahl betrachtet werden, der in der Mitte einer Abstrahlfläche abgestrahlt wird. Bei einer im Wesentlichen punktförmigen Röntgenquelle kann der Zentralstrahl jener Strahl sein, dessen Abstrahlwinkel dem durchschnittlichen Abstrahlwinkel des Strahlenfächers beziehungsweise -konus entspricht.

Durch eine Objekterfassungseinrichtung kann eine Positionsinformation ermittelt werden, die die Position eines an dem Objekt angeordneten Markierelements oder Positionserfassungselements in dem Erfassungsvolumen beschreibt, wobei der Schnittflächenparameter in Abhängigkeit der Positionsinformation ermittelt wird. Eine Positionsinformation kann beispielsweise über eine funkbasierte Triangulation oder durch Sensoren des Positionserfassungselements, die das Magnetfeld erfassen, bestimmt werden. Werden die eingangs genannten aktiven Instrumente genutzt, die Positionsinformationen für wenigstens einen ihrer Punkte bereitstellen, können diese Informationen somit im erfindungsgemäßen Verfahren bei der Bestimmung des Schnittflächenparameters berücksichtigt werden.

Es können zwei der Röntgenaufnahmen unter verschiedenen Aufnahmewinkeln erfasst werden, wonach eine dreidimensionale Position im Erfassungsvolumen für wenigstens einen vorgegebenen Bereich des Objekts aus den Röntgenbildern als Objektinformation bestimmt wird, wonach der Schnittflächenparameter in Abhängigkeit der dreidimensionalen Position ermittelt wird. Insbesondere kann der Schnittflächenparameter derart ermittelt werden, dass die Schnittfläche die dreidimensionale Position umfasst. Es ist auch möglich, dass der Schnittflächenparameter derart vorgegeben wird, dass die Schnittfläche in eine vorgegebene Richtung von der dreidimensionalen Position beabstandet ist. Sind weitere dreidimensionale Positionen im Erfassungsvolumen vorgegeben, beispielsweise eine Zielposition, kann der Schnittflächenparameter derart vorgegeben werden, dass die Schnittfläche eine definierte Orientierung bezüglich einer Verbindungsgerade zwischen diesen Positionen aufweist, insbesondere parallel zu der Verbindungsgerade verläuft, wobei die Verbindungsgerade insbesondere in der Schnittfläche liegen kann, oder senkrecht zu ihr steht.

Es kann eine Röntgeneinrichtung mit mehreren Paaren von Röntgenquellen und Röntgendetektoren verwendet werden, deren Aufnahmewinkel voneinander unterschiedlich sind, wobei die unter den verschiedenen Aufnahmewinkeln erfassten Röntgenbilder durch verschiedene Paare erfasst werden.

Alternativ können die unter den verschiedenen Aufnahmewinkeln aufgenommenen Röntgenaufnahmen durch eine Röntgeneinrichtung aufgenommen werden, die gegenüber dem Erfassungsvolumen rotierbar und/oder verschiebbar gelagert ist. Die Verschiebung beziehungsweise Rotation der Röntgeneinrichtung kann erfasst werden, womit das zur Bildgebung der Röntgeneinrichtung genutzte Koordinatensystem sowohl vor als auch nach der Verschiebung und/oder Rotation zu dem zur Bildgebung der Magnetresonanzeinrichtung genutzten Koordinatensystem registriert sein kann.

Wie bereits erwähnt, kann in dem erfindungsgemäßen Verfahren eine gekrümmte Schnittfläche verwendet werden. Eine Möglichkeit dies zu realisieren ist es, die Messdatenerfassung in einem rechteckigen Abschnitt des Erfassungsvolumens durchzuführen, das die Schnittfläche umfasst. Anschließend kann eine Volumenrekonstruktion von dreidimensionalen Bilddaten erfolgen, wonach die zweidimensionalen Bilddaten entlang der gekrümmten Schnittfläche aus der Volumenrekonstruktion ausgelesen werden. Hierbei können direkt die Daten einzelner dreidimensionaler Voxel ausgelesen werden oder es kann für die einzelnen Bildpunkte zwischen verschiedenen Voxeln interpoliert werden.

Im erfindungsgemäßen Verfahren kann eine Röntgeneinrichtung verwendet werden, die einen Röntgendetektor und eine Röntgenquelle aufweist, deren relative Position und/oder Orientierung bezüglich dem Erfassungsvolumen veränderbar sind, wobei bei einer Benutzerhandlung zur Veränderung der durch die Magnetresonanzeinrichtung erfassten Schnittfläche und/oder bei einer Veränderung des Schnittflächenparameters automatisch die Position und/oder die Orientierung des Röntgendetektors und/oder der Röntgenquelle angepasst werden. Der Röntgendetektor und die Röntgenquelle können vorzugsweise über eine starre Halterung verbunden sein, beispielsweise eine Gantry oder einen C-Arm, um sie gemeinsam zu verschieben und/oder zu rotieren. Die Position und/oder die Orientierung können insbesondere derart angepasst werden, dass die Aufnahmeachse senkrecht zu der durch die Magnetresonanzeinrichtung erfassten Schnittfläche steht.

Neben dem erfindungsgemäßen Verfahren betrifft die Erfindung eine kombiniere Magnetresonanz- und Röntgenvorrichtung mit einer Magnetresonanzeinrichtung, einer Röntgeneinrichtung und einer Steuereinrichtung, wobei die zur Bildgebung genutzten Koordinatensysteme der Röntgeneinrichtung und der Magnetresonanzeinrichtung durch eine mechanische Kopplung der Röntgeneinrichtung mit der Magnetresonanzeinrichtung zueinander registriert sind, wobei die Steuereinrichtung zur Durchführung des erfindungsgemässen Verfahrens konfiguriert ist.

Zudem betrifft die Erfindung ein Computerprogramm, welches direkt in einen Speicher einer Steuereinrichtung einer kombinierten Magnetresonanz- und Röntgenvorrichtung ladbar ist, mit Programm-Mitteln, um die Schritte des erfindungsgemäßen Verfahrens auszuführen, wenn das Computerprogramm in der Steuereinrichtung der kombinierten Magnetresonanz- und Röntgenvorrichtung ausgeführt wird.

Außerdem betrifft die Erfindung einen elektronisch lesbaren Datenträger mit darauf gespeicherten elektronisch lesbaren Steuerinformationen, welche zumindest ein erfindungsgemäßes Computerprogramm umfassen und derart ausgestaltet sind, dass sie bei Verwendung des Datenträgers in einer Steuereinrichtung einer kombinierten Magnetresonanz- und Röntgenvorrichtung das erfindungsgemäße Verfahren durchführen.

Weitere Vorteile und Einzelheiten der Erfindung ergeben sich aus den folgenden Ausführungsbeispielen sowie den zugehörigen Zeichnungen. Dabei zeigen schematisch:
- Fig. 1: ein Ablaufdiagramm eines Ausführungsbeispiels des erfindungsgemäßen Verfahrens,
- Fig. 2: ein in dem Verfahren gemäß Fig. 1 aufgenommenes Röntgenbild mit überlagerter möglicher Schnittfläche,
- Fig. 3: ein Ablaufdiagramm eines weiteren Ausführungsbeispiels des erfindungsgemäßen Verfahrens, und
- Figuren 4 u. 5: verschiedene Ausführungsbeispiele einer erfindungsgemäßen kombinierten Magnetresonanz- und Röntgenvorrichtung.

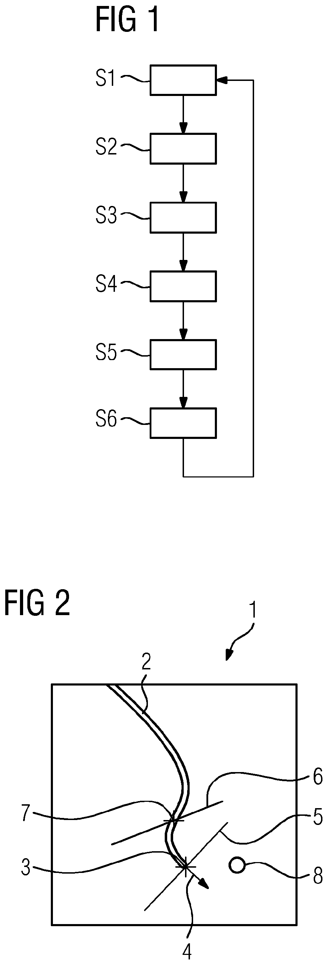

Fig. 1 zeigt ein Ablaufdiagramm eines Ausführungsbeispiels eines Verfahrens zur Ermittlung von zweidimensionalen Bilddaten wenigstens einer Schnittfläche eines Erfassungsvolumens im Rahmen einer Magnetresonanzbildgebung durch eine kombinierte Magnetresonanz- und Röntgenvorrichtung. Die Bildgebung kann der Führung eines Objekts, beispielsweise eines medizinischen Instruments, insbesondere eines Katheters, im Rahmen eines Eingriffs dienen. Hierbei soll eine echtzeitnahe Bildgebung mit hohen Bildwiederholraten für einen Benutzer realisiert werden. Um eine hohe Bildwiederholrate zu erreichen ist es möglich, dass eine Magnetresonanzbildgebung nur für eine oder mehrere Schnittflächen erfolgt. Hierbei sollen die erfassten beziehungsweise dargestellten Schnittflächen derart nachgeführt werden, dass einem Benutzer relevante Schnitte gezeigt werden können, also insbesondere solche Schnitte, in denen die Führung des Objekts zu erkennen ist. Bei einer Bewegung des Objekts soll somit die Schnittfläche möglichst zeitnah nachgeführt werden.

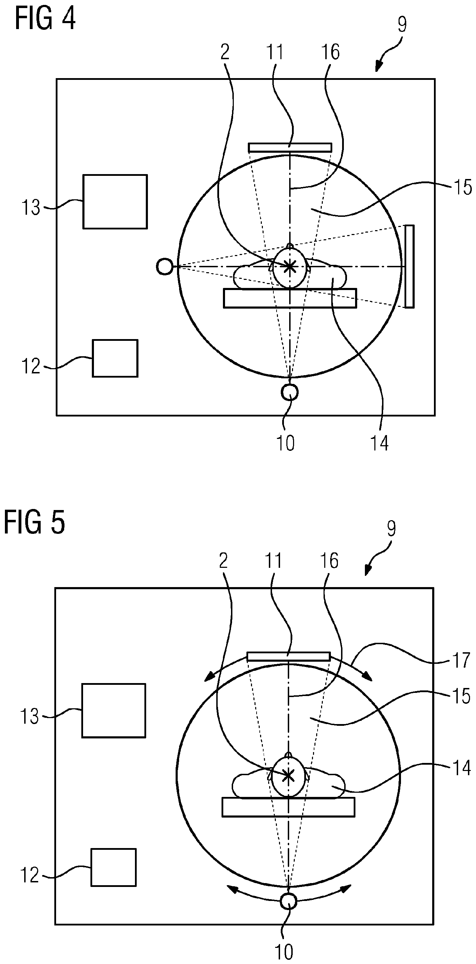

Im erfindungsgemäßen Verfahren wird eine kombinierte Magnetresonanz- und Röntgenvorrichtung genutzt, die eine Magnetresonanzeinrichtung und eine Röntgeneinrichtung umfasst, deren zur Bildgebung genutzte Koordinatensysteme durch eine mechanische Kopplung der Röntgeneinrichtung mit der Magnetresonanzeinrichtung zueinander registriert sind. Derartige kombinierte Magnetresonanz- und Röntgenvorrichtungen werden später mit Bezug auf Fig. 4 und 5 genauer erläutert. Wesentlich für das erfindungsgemäße Verfahren ist, dass aufgrund der bekannten Lage und Ausrichtung der Koordinatensysteme zueinander durch die Röntgeneinrichtung erfasste Merkmale zur Steuerung der Magnetresonanzbildgebung genutzt werden können, da ein definierter Bezug zwischen den Erfassungseinrichtungen besteht.

In Schritt S1 wird die Röntgeneinrichtung zum Erfassen wenigstens eines Röntgenbilds angesteuert, das das Objekt zumindest teilweise abbildet. Dies wird dadurch ermöglicht, dass der Erfassungsbereich der Röntgeneinrichtung derart gewählt wird, dass sich das Objekt in dem Erfassungsbereich befindet.

In Schritt S2 wird eine Bildverarbeitung des Röntgenbildes durchgeführt, um wenigstens eine Objektinformation zu ermitteln, die eine Position und/oder eine Ausrichtung und/oder wenigstens ein Merkmal des Objekts betrifft. Ein derartiges Röntgenbild 1 ist in Fig. 2 beispielhaft dargestellt. Das Röntgenbild 1 zeigt ein Objekt 2, beispielsweise einen in einen Patienten eingeführten Katheter. Die weiteren abgebildeten Objekte, also insbesondere die Knochen des Patienten, sind in Fig. 2 aus Übersichtlichkeitsgründen nicht dargestellt.

Aus dem Röntgenbild 1 sollen zwei Merkmale ermittelt werden, nämlich eine Punktposition 3 einer Katheterspitze und ein Richtungsvektor 4, der eine in die Ebene des Röntgenbildes projizierte Längsrichtung des Objekts 2 im Bereich der Katheterspitze beschreibt. Um die Punktposition 3 zu ermitteln wird das Objekt 2 zunächst in den Bilddaten des Röntgenbilds 1 segmentiert. Hierzu kann eine Vielzahl von aus dem Stand der Technik bekannter Segmentierungsalgorithmen genutzt werden, beispielsweise kann ein Ridge-Detektor genutzt werden und das Objekt 2 kann anschließend durch einen Region-Growing-Algorithmus segmentiert werden. Nach der Segmentierung des Objekts 2 kann das Ende des Objekts 2 in einem Röntgenbild 1 detektiert werden und als die Punktposition 3 bestimmt werden. Alternativ wäre es beispielsweise möglich, die Punktposition 3 durch eine Merkmalsdetektion, beispielsweise auf Basis von skaleninvarianten Merkmalen, zu detektieren. Anhand des segmentierten Objekts 2 kann zudem, beispielsweise durch eine Berechnung einer Mittellinie des Objekts 2, eine Längsrichtung des Objekts 2 im Bereich der Katheterspitze bestimmt werden.

Aus der Punktposition 3 und dem Richtungsvektor 4 werden in Schritt S3 Schichtparameter berechnet, die eine Anordnung einer Schnittfläche 5 in dem Erfassungsvolumen vorgeben. Als Schnittfläche wird eine Schnittebene gewählt, da deren Messdaten im Rahmen der Magnetresonanzbildgebung besonders einfach zu erfassen sind. Die Schnittflächenparameter bestimmen somit eine Position und Orientierung der Schnittebene im Raum. Diese werden so gewählt, dass die Schnittfläche 5 eine Verbindungslinie zwischen dem der Punktposition 3 zugeordneten Röntgendetektorelement und der Röntgenquelle zumindest teilweise umfasst. Hierdurch ist sichergestellt, dass die Katheterspitze in der Schnittebene liegt. In einer alternativen Ausführungsform wäre es möglich, die Schnittflächenparameter derart zu wählen, dass die Schnittebene 5 parallel zu einem Zentralstrahl einer Röntgenquelle liegt, wobei eine Verschiebung der Schnittfläche 5 in der Ebene des Röntgenbildes in Abhängigkeit der Punktposition 3 bestimmt wird. Dies entspricht im Wesentlichen dem vorangehend beschriebenen Vorgehen, wenn eine Röntgenquell mit im Wesentlichen paralleler Röntgenstrahlung genutzt wird oder wenn die Punktposition 3 nahe des Zentrums des Erfassungsbereichs der Röntgeneinrichtung liegt, was beispielsweise dadurch sichergestellt werden kann, dass die Röntgeneinrichtung bei einer Bewegung des Objekts 2 so nachgeführt wird, dass die Katheterspitze und somit die Punktposition 3 jeweils im Zentralbereich des Röntgenbildes 1 liegt.

Die Schnittflächenparameter werden zudem derart gewählt, dass der Richtungsvektor 4 senkrecht auf der Schnittfläche 5 steht. Die resultierenden zweidimensionalen Bilddaten zeigen somit eine Schnittfläche an der momentanen Position der Katheterspitze, die zumindest in der Bildebene des Röntgenbilds senkrecht auf der Katheterspitze steht. Alternativ oder zusätzlich, zur Aufnahme von Messdaten einer weiteren Schnittfläche, wäre es möglich, die Schnittflächenparameter derart vorzugeben, dass eine Punktposition gewählt wird, die in Richtung oder entgegen der Richtung des Richtungsvektors 4 gegenüber der Punktposition 3 verschoben ist, um eine Schnittfläche zu vermessen, die zu der Schnittfläche 5 versetzt ist. Hierdurch kann beispielsweise ein Bereich abgebildet werden, in den das Objekt 2 bei einem weiteren Einführen des Katheters eingeführt wird.

In Schritt S4 wird die Magnetresonanzeinrichtung zur Erfassung von die Schnittfläche betreffenden Messdaten angesteuert. In Schritt S5 erfolgt anschließend eine Berechnung der zweidimensionalen Bilddaten aus den Messdaten, die anschließend in Schritt S6 dargestellt werden. Die Schritte S4 bis S6 entsprechen dem üblichen Vorgehen bei der Erfassung von zweidimensionalen Messdaten durch einen Magnetresonanztomographen und sollen daher nicht detailliert erläutert werden. Wesentlich ist bei dem erläuterten Vorgehen jedoch, dass die Ansteuerung der Magnetresonanzeinrichtung in Schritt S4 in Abhängigkeit der Schnittflächenparameter folgt, so dass Messdaten der Schnittfläche 5 erfasst werden.

In einigen Fällen kann es gewünscht sein, zweidimensionale Bilddaten einer gekrümmten Messfläche abzubilden. Beispielsweise könnte der Verlauf des Katheters 2 ermittelt werden und die Schnittfläche könnte entlang dem in Fig. 1 gezeigten gekrümmten Verlauf des Objekts 2 geführt werden. Dies ist beispielsweise dadurch möglich, dass in Schritt S4 die Magnetresonanzeinrichtung derart angesteuert wird, dass Messdaten eines rechteckigen Volumens erfasst werden, das die Schnittfläche umfasst. Es wird somit eine Volumenbildgebung auf einem potentiell reduzierten Erfassungsvolumen durchgeführt. Aus diesen Messdaten kann entsprechend dem üblichen Vorgehen bei der Volumenbildgebung im Magnetresonanztomographen ein dreidimensionaler Bilddatensatz generiert werden, aus dem gemäß der vorgegebenen gekrümmten Schnittfläche bestimmte Messpunkte ausgelesen oder interpoliert werden, um die zweidimensionalen Bilddaten zu berechnen.

Wie in Fig. 1 dargestellt, werden die Schritte S1 bis S6 wiederholt durchgeführt, um eine Bildgebung zu ermöglichen, die nahezu in Echtzeit erfolgt. Es ist hierbei möglich, dass bei späteren Wiederholungen der Schnittflächenparameter in Schritt S3 in Abhängigkeit von den während früherer Wiederholungen in Schritt S4 erfassten Messdaten ermittelt wird. Ist es beispielsweise gewünscht, die Schnittfläche 5 anders zu orientieren, wobei sie weiterhin die Katheterspitze umfassen soll, so ist dies bei einer ausschließlichen Bestimmung der Schnittflächenparameter aus dem Röntgenbild 1 beziehungsweise der Objektinformation nicht in allen Fällen möglich, da das Röntgenbild 1 keine Tiefeninformation für die Punktposition 3 bereitstellt. Eine entsprechende Tiefeninformation kann jedoch durch Berücksichtigung der vorangehend erfassten Messdaten bestimmt werden. Beispielsweise kann in den in Schritt S5 der vorangehenden Wiederholung ermittelten zweidimensionalen Bilddaten jene Position ermittelt werden, an der das Objekt 2 die Schnittfläche 5 schneidet. Da die Lage der Schnittfläche 5 im Erfassungsvolumen bekannt ist, kann hieraus eine dreidimensionale Position des Schnittpunkts und somit des Objekts 2 im Bereich der Schnittfläche 5 ermittelt werden. Daher können die Schnittflächenparameter in der darauffolgenden Wiederholung derart gewählt werden, dass diese dreidimensionale Position innerhalb der Schnittfläche 5 liegt.

Es kann gewünscht sein, zweidimensionale Bilddaten einer Schnittfläche zu berechnen und anzuzeigen, die derart gewählt ist, dass das Objekt zumindest im Bereich seines Endes, also beispielsweise der Katheterspitze, innerhalb dieser Schnittfläche verläuft. Zusätzlich kann es gewünscht sein, dass in dieser Schnittfläche auch ein Zielpunkt 8 abgebildet wird. Eine entsprechende Bildgebung kann durch eine geringfügige Modifikation des zu Fig. 1 erläuterten Verfahrens erreicht werden. Hierbei werden, wie vorangehend erläutert, die in einer vorangehenden Wiederholung ermittelten Messdaten berücksichtigt, um einen Schnittpunkt 2 des Objekts mit der Schnittfläche 5 der vorangehenden Wiederholung und somit eine dreidimensionale Position des Objekts 2 zu ermitteln. Um eine Schnittfläche aufzufinden, in der das Objekt 2 im relevanten Bereich liegt, soll eine weitere dreidimensionale Position des Objekts 2 ermittelt werden. Dies wird dadurch erreicht, dass vor der Ermittlung des Schnittflächenparameters in Schritt S3 die Magnetresonanzeinrichtung zur Erfassung einer Hilfsschnittfläche 6 angesteuert wird. Die Hilfsschnittfläche 6 wird in Abhängigkeit der Objektinformation derart bestimmt, dass sie das Objekt 2 schneidet. Die Hilfsschnittfläche 6 ist zudem von der Schnittfläche 5, deren Messdaten in der früheren Wiederholung erfasst wurden, beabstandet und steht gewinkelt zu dieser. Wie vorangehend zur Schnittfläche 5 erläutert, wird nun ein Schnittpunkt des Objekts 2 mit der Hilfsschnittfläche 6, also der Hilfsschnittpunkt 7, bestimmt.

Es sind somit zwei dreidimensionale Positionen im Erfassungsvolumen bekannt, durch die die Schnittfläche verlaufen soll, nämlich der Schnittpunkt des Objekts 2 mit der Schnittfläche 5 und der Hilfsschnittpunkt 7 des Objekts 2 mit der Hilfsschnittfläche 6. Ist zusätzlich die Zielposition 8 als dreidimensionale Zielposition vorgegeben, so kann in Schritt S3 der Schichtparameter derart vorgegeben werden, dass die Schnittfläche, deren Messdaten in Schritt S4 erfasst werden, alle drei dieser Punkte umfasst. Somit können bei einer Führung eines Katheters beispielsweise der Katheter im Bereich seiner Spitze sowie der Ort, zu dem der Katheter geführt werden soll, innerhalb einer Schnittfläche, insbesondere einer Schnittebene, visualisiert werden.

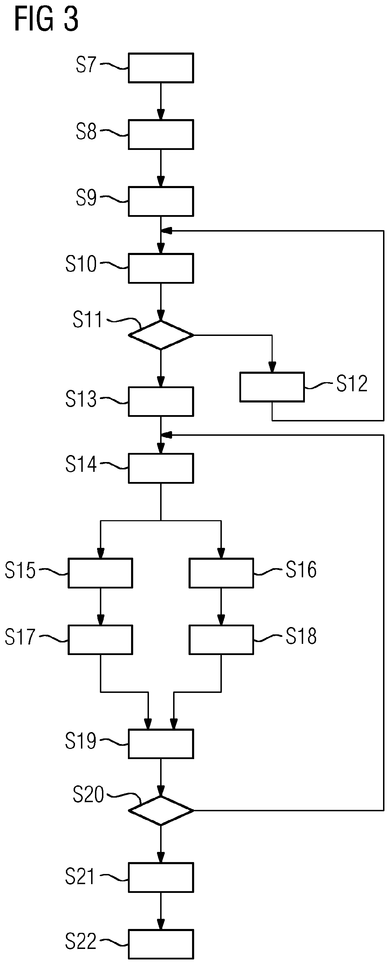

Fig. 3 zeigt ein Ablaufdiagramm eines weiteren Ausführungsbeispiels eines Verfahrens zur Ermittlung von zweidimensionalen Bilddaten wenigstens einer Schnittfläche eines Erfassungsvolumens im Rahmen einer Magnetresonanzbildgebung. Wie zu Fig. 1 erläutert, soll die Bildgebung mit Bezug auf ein Objekt erfolgen. Die Schritte S7 bis S10 entsprechen den Schritten S1 bis S4 in dem zu Fig. 1 erläuterten Verfahren, wobei in Schritt S7 ein Röntgenbild erfasst wird, in Schritt S8 eine Bildverarbeitung dieses Röntgenbilds durchgeführt wird, um Objektinformationen zu bestimmen und in Schritt S9 aus diesen Objektinformationen Schnittflächenparameter bestimmt werden, in Abhängigkeit deren in Schritt S10 Messdaten der durch die Schnittflächenparameter parametrisierten Schnittfläche erfasst werden.

In Schritt S11 wird ermittelt, ob die Messdaten das Objekt zumindest teilweise abbilden. Dies kann beispielsweise durch eine Merkmalsdetektion in den Messdaten beziehungsweise in aus den Messdaten berechneten zweidimensionalen Bilddaten erfolgen. Ist dies nicht der Fall, so werden in Schritt S12 neue Schnittflächenparameter bestimmt, in dem die bisherigen Schichtflächenparameter variiert werden, wonach das Verfahren ab Schritt S10 wiederholt wird. Die Variation der Schichtparameter kann nach einem fest vorgegebenen Muster erfolgen, das beispielsweise eine Abfolge von Orientierungs- und/oder Positionsänderungen der Schnittfläche beschreibt. Vorzugsweise erfolgt die Variation jedoch in Abhängigkeit der in Schritt S8 ermittelten Objektinformation, wobei beispielsweise der Schnittflächenparameter derart variiert werden kann, dass die Schnittfläche in oder entgegen einer ermittelten Längsrichtung des Objekts verschoben wird.

Wurde in Schritt S11 ermittelt, dass die Messdaten das Objekt zumindest teilweise abbilden, so erfolgt in den Schritten S13 bis S20 eine Optimierung der Abbildung derart, dass der Anteil des Objekts, der durch die Messdaten abgebildet wird, maximiert wird. Dies erfolgt in dem Ausführungsbeispiel durch ein Gradientenverfahren, wobei auch andere Optimierungsverfahren nutzbar sind.

In Schritt S14 wird für jeden Freiheitsgrad, also jeden der Schnittflächenparameter, eine Variation durchgeführt, wobei für jeden Schnittflächenparameter vorzugsweise zwei neue Werte generiert werden, von denen einer etwas größer und einer etwas kleiner ist als der vorangehende Wert. Die Anzahl der generierten Parametersätze ist, wenn alle Schnittflächenparameter und somit alle Freiheitsgrade gleichzeitig variiert werden, 2ⁿ, wobei n die Anzahl der Schnittflächenparameter beziehungsweise der Freiheitsgrade ist. Werden viele Schnittflächenparameter genutzt, ist es möglich, die Variation auf einige dieser Parameter einzuschränken, um den resultierenden Mess- und Verarbeitungsaufwand zu reduzieren und somit eine höhere Bildwiederholrate im beschriebenen Verfahren zu erreichen. Aus Übersichtlichkeitsgründen ist die beschriebene Variation in Fig. 3 nur für einen Freiheitsgrad dargestellt, womit in den Schritten S15 bis S18 nur zwei Zweige resultieren.

In den Schritten S15 und S16 werden jeweils Messdaten für eine Schnittfläche erfasst, die durch die jeweiligen in Schritt S14 generierten Werte für den Schnittflächenparameter parametrisiert ist. Je nach Lage dieser Schnittflächen und der konkreten Ausgestaltung der Magnetresonanzeinrichtung kann es erforderlich sein, dass die Erfassung der Messdaten für die verschiedenen Schnittflächen in den Schritten S15 und S16 zeitlich nacheinander erfolgt.

In den Schritten S17 und S18 wird für die in Schritt S15 beziehungsweise in Schritt S16 ermittelten Messdaten jeweils eine Abbildungsinformation ermittelt, die beschreibt, welcher Anteil des Objekts durch die Messdaten abgebildet wird. Die Abbildungsinformation kann beispielsweise dadurch ermittelt werden, dass aus den Messdaten jeweils zweidimensionale Bilddaten berechnet werden, in denen das Objekt segmentiert wird, wonach die Fläche des dem Objekt zugeordneten Segments als Abbildungsinformation bereitgestellt wird. In einer alternativen Ausführungsform kann eine Segmentierung des Objekt vorgegeben werden, wonach ermittelt wird, wie viele der Segmente in den jeweiligen Messdaten enthalten sind, wonach diese Anzahl der Segmente als Abbildungsinformation bereitgestellt wird. Die Segmentierung des Objekts kann als Vorwissen, beispielsweise aus einer Datenbank, die medizinische Instrumente beschreibt, bereitgestellt werden. Es ist jedoch auch möglich, diese Segmentierung im Röntgenbild durchzuführen. Eine Segmentierung im Röntgenbild kann hierbei zusätzlich Vorwissen nutzen, beispielsweise indem das Röntgenbild elastisch oder starr zu einem dreidimensionalen Datensatz registriert wird, der das Objekt beschreibt. Eine Erkennung der Segmente des Objekts in den Messdaten beziehungsweise in den aus diesen berechneten Bilddaten kann durch eine Merkmalserkennung erfolgen. Hierbei können die unterschiedlichen Segmente unterschiedliche oder gleiche Merkmale aufweisen, da ausschließlich die Anzahl der Segmente, die durch die Messdaten abgebildet werden, ausgewertet wird.

Eine weitere Möglichkeit, die Abbildungsinformation zu bestimmen, ist es, Verfahren des Maschinenlernens zu nutzen. Beispielsweise kann ein Algorithmus mit einer Vielzahl von Trainingsdatensätzen trainiert werden, die jeweils Anteile des Objekts abbilden. Durch manuelle oder automatische Annotierung dieser Trainingsdaten kann jeweils eine Information hinzugefügt werden, welcher Anteil des Objekts in dem jeweiligen Trainingsdatensatz abgebildet wird beziehungsweise welchen Wert die Abbildungsinformation für den entsprechenden Trainingsdatensatz aufweist. Mit diesen Trainingsdaten kann ein Algorithmus trainiert werden, um anschließend für Messdaten bestimmen zu können, welche Abbildungsinformation diesen jeweils zuzuordnen ist.

Im Schritt S19 wird anschließend jener Satz an Messdaten mit den zugehörigen Schnittflächenparameter ausgewählt, für den in den Schritten S17 beziehungsweise S18 eine Abbildungsinformation ermittelt wurde, die eine Abbildung eines größeren Objektanteils beschreibt. In Schritt S20 wird anschließend ermittelt, ob bereits in einer vorangehenden Wiederholung der Schritte S14 bis S19 eine Abbildungsinformation ermittelt wurde und ob die neue Abbildungsinformation um einen Wert von dieser abweicht, der kleiner als ein Grenzwert ist. Ist dies der Fall, so wird davon ausgegangen, dass das in den Schritten S14 bis S20 durchgeführte Optimierungsverfahren konvergiert ist, das heißt, dass zumindest ein lokales Maximum des Anteils des Objekts, dass durch die Messdaten abgebildet wird, erreicht wurde. Ist dies der Fall, so werden in Schritt S21 aus den in Schritt S19 gewählten Messdaten zweidimensionale Bilddaten generiert und diese in Schritt S22 dargestellt.

Wurde vorangehend noch keine Abbildungsinformationen ermittelt oder ist der Unterschied zwischen den aufeinanderfolgenden Abbildungsinformationen zu groß, so wird das Verfahren ab Schritt S14 fortgesetzt, da davon ausgegangen wird, dass ein optimaler Anteil der Abbildung des Objekts noch nicht erreicht wurde.

Fig. 4 zeigt ein Ausführungsbeispiel einer kombinierten Magnetresonanz- und Röntgenvorrichtung mit einer Steuereinrichtung 12, die dazu eingerichtet sein kann, das mit Bezug auf Fig. 1 und/oder das mit Bezug auf Fig. 3 erläuterte Verfahren durchzuführen. Hierzu steuert die Steuereinrichtung 12 eine Magnetresonanzeinrichtung, die mehrere Spulen zur Erzeugung von Magnetfeldern umfasst, die jedoch aus Übersichtlichkeitsgründen nicht dargestellt sind. Zudem steuert die Steuereinrichtung 12 eine Röntgeneinrichtung, die die Röntgenquellen 10 und die Röntgendetektoren 11 umfasst. Die Röntgenquellen 10 strahlen Röntgenstrahlung mit einer konusförmigen Strahlgeometrie 15 ab, die im Wesentlichen rotationssymmetrisch um einen Zentralstrahl 16 ist. Die Röntgenquellen 10 und die Röntgendetektoren 11 sind starr mit den Komponenten der Magnetresonanzeinrichtung gekoppelt, womit die zur Bildgebung genutzten Koordinatensysteme der Röntgeneinrichtung und der Magnetresonanzeinrichtung zueinander registriert sind beziehungsweise ein gemeinsames Koordinatensystem genutzt werden kann. Die kombinierte Magnetresonanz- und Röntgenvorrichtung 9 umfasst zudem eine Anzeigevorrichtung 13, durch die die zweidimensionalen Bilddaten, wie zu Fig. 1 und 3 erläutert, ausgegeben werden können.

Da zwei Röntgenquellen 10 und zwei Röntgendetektoren 11 vorhanden sind, die das Objekt 2, das aus Übersichtlichkeitsgründen als Kreuz dargestellt ist und das in einen Patienten 14 eingeführt ist, aus zwei Aufnahmewinkeln erfassen, kann ergänzend oder alternativ zu den in Fig. 1 und Fig. 3 erläuterten Verfahren auch ein weiteres Verfahren zur Ermittlung von zweidimensionalen Bilddaten durchgeführt werden. Hierbei werden zwei Röntgenaufnahmen unter verschiedenen Aufnahmewinkeln erfasst, wodurch eine dreidimensionale Position im Erfassungsvolumen für wenigstens einen vorgegebenen Bereich des Objekts 2 aus den Röntgenbildern als Objektinformation bestimmt wird. Beispielsweise kann direkt eine dreidimensionale Position der in Fig. 2 dargestellten Katheterspitze bestimmt werden. Anschließend wird der Schnittflächenparameter in Abhängigkeit der dreidimensionalen Position ermittelt. Der Schnittflächenparameter kann derart ermittelt werden, dass die dreidimensionale Position innerhalb der Schnittfläche liegt oder die Schnittfläche kann beliebig bezüglich der dreidimensionalen Position verschoben und/oder verschwenkt werden. Aus den Röntgenaufnahmen unter unterschiedlichen Aufnahmewinkeln können auch mehrere dreidimensionale Positionen ermittelt werden, die beispielsweise alle innerhalb der Schnittfläche liegen sollen.

Ein weiteres Ausführungsbeispiel einer kombinierten Magnetresonanz- und Röntgenvorrichtung 9 ist in Fig. 5 gezeigt. Der Aufbau der Magnetresonanz- und Röntgenvorrichtung 9 entspricht im Wesentlichen dem mit Bezug auf Fig. 4 erläuterten Aufbau. Der wesentliche Unterschied ist, dass nur eine Röntgenquelle 10 und ein Röntgendetektor 11 vorgesehen sind. Die Röntgenquelle 10 und der Röntgendetektor 11 können, wie zu Fig. 4 erläutert, starrer mit den Komponenten der Magnetresonanzeinrichtung gekoppelt sein. Dies ermöglicht es beispielsweise, die mit Bezug auf Fig. 1 und Fig. 3 erläuterten Ausführungsbeispiele des Verfahrens durchzuführen, da diese ausschließlich Röntgenaufnahmen aus einer einzigen Perspektive erfordern. Es ist jedoch auch möglich, dass die Röntgenquelle 10 und der Röntgendetektor 11, wie durch die Pfeile 17 angedeutet, gemeinsam um das Erfassungsvolumen rotiert werden können. Dies kann insbesondere automatisch durch einen nicht gezeigten Aktor erfolgen, der eine nicht gezeigte Gantry, an der die Röntgenquelle 10 und der Röntgendetektor 11 befestigt sind, um das Erfassungsvolumen rotiert. Der Grad der Rotation kann hierbei durch die Steuereinrichtung 12 erfasst oder gesteuert werden, so dass in der Steuereinrichtung 12 trotz der möglichen Rotation stets eine Registrierung zwischen den zur Bildgebung genutzten Koordinatensysteme und der Röntgeneinrichtungen und der Magnetresonanzeinrichtung bekannt ist. Wird eine derartige Röntgenaufnahmeeinrichtung genutzt, die eine Aufnahme von Röntgenaufnahmen unter verschiedenen Aufnahmewinkeln ermöglicht, kann auch die mit Bezug auf Fig. 4 erläuterte Verfahrensvariante durchgeführt werden, bei der aus mehreren mit unterschiedlichen Aufnahmewinkeln erfasste Röntgenaufnahmen wenigstens eine dreidimensionale Position ermittelt wird, in deren Abhängigkeit der Schnittflächenparameter bestimmt wird. Hierbei kann es ausreichend sein, den Aufnahmewinkel um einen relativ kleinen Winkel, beispielsweise fünf Grad oder zehn Grad, zu ändern. Insbesondere kann der Aufnahmewinkel zwischen zwei definierten Winkeln hin und her geschwenkt werden, wobei an den beiden Umkehrpunkten der Rotation jeweils ein Röntgenbild aufgenommen wird.

Ein hierin beschriebenes Verfahren kann auch in Form eines Computerprogramms vorliegen, das das Verfahren auf einer Steuereinrichtung 12 implementiert, wenn es auf der Steuereinrichtung 12 ausgeführt wird. Ebenso kann ein nicht dargestellter elektronisch lesbarer Datenträger mit darauf gespeicherten elektronisch lesbaren Steuerinformationen vorliegen, welche zumindest ein beschriebenes Computerprogramm umfassen und derart ausgestaltet sind, dass sie bei Verwendung des Datenträgers in der Steuereinrichtung 12 der kombinierten Magnetresonanz- und Röntgenvorrichtung 9 das beschriebene Verfahren durchführen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Ermittlung von zweidimensionalen Bilddaten wenigstens einer Schnittfläche (5) eines Erfassungsvolumens, im Rahmen einer Magnetresonanzbildgebung durch eine kombinierte Magnetresonanz- und Röntgenvorrichtung (9), die eine Magnetresonanzeinrichtung und eine Röntgeneinrichtung umfasst, deren zur Bildgebung genutzte Koordinatensysteme durch eine mechanische Kopplung der Röntgeneinrichtung mit der Magnetresonanzeinrichtung zueinander registriert sind, umfassend die durch eine Steuereinrichtung (12) durchgeführten Schritte:
- Ansteuern der Röntgeneinrichtung zum Erfassen wenigstens eines Röntgenbilds (1), das ein Objekt (2), insbesondere ein medizinisches Instrument, zumindest teilweise abbildet,
- Ermitteln wenigstens einer Objektinformation, die eine Position und/oder eine Ausrichtung und/oder wenigstens ein Merkmal des Objekts (2) betrifft, durch eine Bildverarbeitung des Röntgenbilds (1),
- Ermitteln wenigstens eines Schnittflächenparameters, der eine Anordnung der Schnittfläche (5) in dem Erfassungsvolumen vorgibt, in Abhängigkeit der Objektinformation,
- Ansteuern der Magnetresonanzeinrichtung zur Erfassung von die Schnittfläche (5) betreffenden Messdaten, und
- Berechnen der zweidimensionalen Bilddaten aus den Messdaten,
wobei das Ansteuern der Magnetresonanzeinrichtung und/oder die Berechnung der zweidimensionalen Bilddaten in Abhängigkeit des Schnittflächenparameters erfolgt.

2. Verfahren nach Anspruch 1, **dadurch gekennzeichnet, dass** als Objektinformation eine jeweilige Punktposition (3) wenigstens eines Messpunktes in dem Röntgenbild (1) ermittelt wird, der in einer vorgegebenen Position bezüglich der zumindest teilweisen Abbildung des Objekts (2) liegt, wonach der Schnittflächenparameter in Abhängigkeit der Punktposition (3) oder der Punktpositionen (3) derart ermittelt wird, dass die Schnittfläche (5) zumindest einen Abschnitt einer jeweiligen Verbindungslinie zwischen einem dem jeweiligen Messpunkt zugeordneten Röntgendetektorelement und einer Röntgenquelle (10) der Röntgeneinrichtung umfasst.

3. Verfahren nach Anspruch 1 oder 2, **dadurch gekennzeichnet, dass** als Objektinformation wenigstens ein Richtungsvektor (4) in dem Röntgenbild (1) ermittelt wird, der eine bezüglich der zumindest teilweisen Abbildung des Objekts (2) vorgegebene Richtung beschreibt, wonach der Schnittflächenparameter in Abhängigkeit des Richtungsvektors (4) oder der Richtungsvektoren (4) derart ermittelt wird, dass zumindest ein jeweiliger Abschnitt der Schnittfläche (5) in dem Erfassungsraum parallel zu dem jeweiligen Richtungsvektor oder in einem vorgegebenen Winkel zu diesem steht.

4. Verfahren nach Anspruch 2 oder Anspruch 3, sofern von Anspruch 2 abhängig, **dadurch gekennzeichnet, dass** das Objekt (2) ein Katheter ist, wobei die Position einer Katheterspitze in dem Röntgenbild (1) ermittelt wird und der wenigstens eine Messpunkt an der Position der Katheterspitze oder an einer bezüglich der Position der Katheterspitze vorgegebenen weiteren Position liegt, und/oder wobei der Richtungsvektor (4) in eine Längsrichtung des Katheters gerichtet ist, die aus dem Röntgenbild (1) ermittelt wird.

5. Verfahren nach einem der vorangehenden Ansprüche,
**dadurch gekennzeichnet, dass** eine Gruppe der Schritte, die zumindest die Ermittlung des jeweiligen Schnittflächenparameters und die Ansteuerung der Magnetresonanzeinrichtung zur Erfassung der die jeweilige Schnittfläche (5) betreffenden Messdaten umfasst, zeitlich aufeinanderfolgend wiederholt durchgeführt wird, wobei in wenigstens einer der Wiederholungen der jeweilige Schnittflächenparameter in Abhängigkeit von den in einer jeweiligen früheren Wiederholung erfassten Messdaten ermittelt wird.

6. Verfahren nach Anspruch 5, **dadurch gekennzeichnet, dass** durch die in der früheren Wiederholung erfassten Messdaten das Objekt (2) zumindest teilweise abgebildet wird, wobei durch eine Verarbeitung dieser Messdaten eine weitere Objektinformation bestimmt wird, die die Position und/oder die Ausrichtung und/oder das oder wenigstens ein weiteres Merkmal des Objekts (2) betrifft, wobei der Schnittflächenparameter in Abhängigkeit der weiteren Objektinformation ermittelt wird.

7. Verfahren nach Anspruch 6, **dadurch gekennzeichnet, dass** die weitere Objektinformation einen Schnittpunkt des Objekts mit der Schnittfläche (5) beschreibt.

8. Verfahren nach Anspruch 7, **dadurch gekennzeichnet, dass** während wenigstens einer der Wiederholungen vor dem Ermitteln des Schnittflächenparameters die Magnetresonanzeinrichtung zur Erfassung von eine Hilfsschnittfläche (6) betreffenden Hilfsmessdaten angesteuert wird, wobei die Hilfsschnittfläche (6) von jener Schnittfläche (5), deren Messdaten in der früheren Wiederholung erfasst werden, beabstandet ist und/oder in einem vorgegebenen Winkel zu dieser steht, wonach ein Hilfsschnittpunkt (7) des Objekts (2) mit der Hilfsschnittfläche (6) bestimmt wird, wonach der Schnittflächenparameter derart ermittelt wird, dass die Schnittfläche den Schnittpunkt und den Hilfsschnittpunkt (7) umfasst oder einen vorgegebenen Winkel mit einer Verbindungslinie zwischen dem Schnittpunkt und dem Hilfsschnittpunkt (7) einschließt.

9. Verfahren nach einem der vorangehenden Ansprüche, **dadurch gekennzeichnet, dass** die oder eine Gruppe von Schritten, die zumindest die Ermittlung des jeweiligen Schnittflächenparameters und die Ansteuerung der Magnetresonanzeinrichtung zur Erfassung der jeweiligen die jeweilige Schnittfläche (5) betreffenden Messdaten umfasst, wiederholt wird, wenn nach dem Erfassen der Messdaten ermittelt wird, dass die Messdaten das Objekt (2) nicht abbilden, wobei der Schnittflächenparameter gemäß einem fest oder in Abhängigkeit der Objektinformation vorgegebenen Muster variiert wird.

10. Verfahren nach einem der vorangehenden Ansprüche,
**dadurch gekennzeichnet, dass**, wenn die Messdaten das Objekt (2) zumindest teilweise abbilden, durch eine Verarbeitung der Messdaten eine Abbildungsinformation ermittelt wird, die beschreibt, welcher Anteil des Objekts (2) durch die Messdaten abgebildet wird, wonach bei Erfüllung einer die Abbildungsinformation auswertenden Wiederholungsbedingung die oder eine Gruppe der Schritte wiederholt wird, die zumindest die Ermittlung des jeweiligen Schnittflächenparameters und die Ansteuerung der Magnetresonanzeinrichtung zur Erfassung der jeweiligen die jeweilige Schnittfläche (5) betreffenden Messdaten umfasst, wobei der Schnittflächenparameter gemäß einem fest oder in Abhängigkeit von der Objektinformation und/oder den Messdaten vorgegebenen Muster variiert wird, und bei Nichterfüllung der Wiederholungsbedingung die zweidimensionalen Bilddaten in Abhängigkeit der vorangehend erfassten Messdaten berechnet werden.

11. Verfahren nach Anspruch 10, **dadurch gekennzeichnet, dass** die Abbildungsinformation in Abhängigkeit wenigstens eines Merkmals des Objekts (2) ermittelt wird, das durch die Objektinformation und/oder ein Vorwissen über das Objekt (2) beschrieben oder daraus ermittelt wird.

12. Verfahren nach Anspruch 10 oder 11, **dadurch gekennzeichnet, dass** als Abbildungsinformation ein Maß dafür bestimmt wird, welcher Anteil des Objekts (2) durch die Messdaten abgebildet wird, wobei die Wiederholungsbedingung die Abbildungsinformation mit einem vorgegebenen Grenzwert vergleicht oder ein Konvergenzkriterium auswertet, das mehrere in aufeinanderfolgenden Wiederholungen der Gruppe der Schritte ermittelte Abbildungsinformationen berücksichtigt.

13. Verfahren nach einem der vorangehenden Ansprüche,
**dadurch gekennzeichnet, dass** ein Zielpunkt (8) in dem Erfassungsvolumen vorgegeben wird, wobei der Schnittflächenparameter derart ermittelt wird, dass die Schnittfläche den Zielpunkt (8) umfasst.

14. Verfahren nach einem der vorangehenden Ansprüche,
**dadurch gekennzeichnet, dass** die Schnittfläche (5) eine Schnittebene ist, wobei die Schnittfläche (5) parallel zu einem Zentralstrahl (16) einer Röntgenquelle (10) der Röntgeneinrichtung liegt.

15. Verfahren nach einem der vorangehenden Ansprüche,
**dadurch gekennzeichnet, dass** durch eine Objekterfassungseinrichtung eine Positionsinformation ermittelt wird, die die Position eines an dem Objekt (2) angeordneten Markierelements oder Positionserfassungselements in dem Erfassungsvolumen beschreibt, wobei der Schnittflächenparameter in Abhängigkeit der Positionsinformation ermittelt wird.

16. Verfahren nach einem der vorangehenden Ansprüche,
**dadurch gekennzeichnet, dass** zwei der Röntgenbilder (1) unter verschiedenen Aufnahmewinkeln erfasst werden, wonach eine dreidimensionale Position im Erfassungsvolumen für wenigstens einen vorgegebenen Bereich des Objekts (2) aus den Röntgenbildern (1) als Objektinformation bestimmt wird, wonach der Schnittflächenparameter in Abhängigkeit der dreidimensionalen Position ermittelt wird.

17. Verfahren nach Anspruch 16, **dadurch gekennzeichnet, dass** eine Röntgeneinrichtung mit mehreren Paaren von Röntgenquellen (10) und Röntgendetektoren (11) verwendet wird, deren Aufnahmewinkel voneinander unterschiedlich sind, wobei die unter den verschiedenen Aufnahmewinkeln erfassten Röntgenbilder (1) durch verschiedene Paare erfasst werden.

18. Verfahren nach Anspruch 16, **dadurch gekennzeichnet, dass** die unter den verschiedenen Aufnahmewinkeln aufgenommenen Röntgenbilder (1) durch eine Röntgeneinrichtung aufgenommen werden, die gegenüber dem Erfassungsvolumen rotierbar und/oder verschiebbar gelagert ist.

19. Verfahren nach einem der vorangehenden Ansprüche,
**dadurch gekennzeichnet, dass** eine gekrümmte Schnittfläche verwendet wird.

20. Verfahren nach einem der vorangehenden Ansprüche,
**dadurch gekennzeichnet, dass** eine Röntgeneinrichtung verwendet wird, die einen Röntgendetektor (11) und eine Röntgenquelle (10) aufweist, deren relative Position und/oder Orientierung bezüglich dem Erfassungsvolumen veränderbar sind, wobei bei einer Benutzerhandlung zur Veränderung der durch die Magnetresonanzeinrichtung erfassten Schnittfläche und/oder bei einer Veränderung des Schnittflächenparameters automatisch die Position und/oder die Orientierung des Röntgendetektors (11) und/oder der Röntgenquelle (10) angepasst werden.

21. Kombinierte Magnetresonanz- und Röntgenvorrichtung mit einer Magnetresonanzeinrichtung, einer Röntgeneinrichtung und einer Steuereinrichtung (12), wobei die zur Bildgebung genutzten Koordinatensysteme der Röntgeneinrichtung und der Magnetresonanzeinrichtung durch eine mechanische Kopplung der Röntgeneinrichtung mit der Magnetresonanzeinrichtung zueinander registriert sind, wobei die Steuereinrichtung (12) zur Durchführung des Verfahrens gemäß einem der vorangehenden Ansprüche konfiguriert ist.

22. Computerprogramm, welches direkt in einen Speicher einer Steuereinrichtung (12) einer kombinierten Magnetresonanz- und Röntgenvorrichtung (9) ladbar ist, mit Programm-Mitteln, um die Schritte des Verfahrens nach einem der Ansprüche 1 bis 20 auszuführen, wenn das Computerprogramm in der Steuereinrichtung (12) der kombinierten Magnetresonanz- und Röntgenvorrichtung (13) ausgeführt wird.

23. Elektronisch lesbarer Datenträger mit darauf gespeicherten elektronisch lesbaren Steuerinformationen, welche zumindest ein Computerprogramm nach Anspruch 22 umfassen und derart ausgestaltet sind, dass sie bei Verwendung des Datenträgers in einer Steuereinrichtung (12) einer kombinierten Magnetresonanz- und Röntgenvorrichtung (9) das Verfahren nach einem der Ansprüche 1 bis 20 durchführen.

## Claims

1. Method for determining two-dimensional image data from at least one sectional surface (5) of an acquisition volume as part of a magnetic resonance imaging process by a combined magnetic resonance imaging and X-ray apparatus (9) comprising a magnetic resonance imaging facility and an X-ray facility, wherein the coordinate systems thereof that are used for imaging are registered to each other by a mechanical coupling of the X-ray facility to the magnetic resonance imaging facility, which method comprises the steps, which are performed by a control facility (12):
- controlling the X-ray facility to acquire at least one X-ray image (1), which images at least part of an object (2), in particular a medical instrument;
- determining at least one piece of object information, which relates to a position and/or an orientation and/or at least one feature of the object (2), by image processing of the X-ray image (1);
- determining on the basis of the object information at least one sectional-surface parameter, which defines an arrangement of the sectional surface (5) in the acquisition volume;
- controlling the magnetic resonance imaging facility to acquire measurement data relating to the sectional surface (5); and
- calculating the two-dimensional image data from the measurement data,
wherein the sectional-surface parameter is used as the basis for said control of the magnetic resonance imaging facility and/or for said calculation of the two-dimensional image data.

2. Method according to claim 1, **characterised in that** an associated point position (3) of at least one measurement point is determined in the X-ray image (1) as the object information, which point lies in a defined position with respect to the at least partial image of the object (2), whereby the sectional-surface parameter is determined on the basis of the point position (3) or the point positions (3) such that the sectional surface (5) comprises at least one segment of a corresponding connecting line between an X-ray detector element associated with the particular measurement point and an X-ray source (10) of the X-ray facility.

3. Method according to claim 1 or 2, **characterised in that** at least one direction vector (4) is determined in the X-ray image (1) as the object information, which vector specifies a direction defined with respect to the at least partial image of the object (2), whereby the sectional-surface parameter is determined on the basis of the direction vector (4) or direction vectors (4) such that at least one corresponding segment of the sectional surface (5) in the acquisition space lies parallel to the corresponding direction vector or at a defined angle thereto.

4. Method according to claim 2 or claim 3, when dependent upon claim 2, **characterised in that** the object (2) is a catheter, wherein the position of a catheter tip is determined in the X-ray image (1), and the at least one measurement point lies at the position of the catheter tip or at another position defined with respect to the position of the catheter tip, and/or where the direction vector (4) is directed in a longitudinal direction of the catheter, which direction is determined from the X-ray image (1).

5. Method according to one of the preceding claims, **characterised in that** a group of the steps that comprises at least determining the particular sectional-surface parameter and controlling the magnetic resonance imaging facility to acquire the measurement data relating to the particular sectional surface (5) is repeated successively in time, wherein in at least one of the repetitions, the particular sectional-surface parameter is determined on the basis of the measurement data acquired in a corresponding earlier repetition.

6. Method according to claim 5, **characterised in that** the measurement data acquired in the earlier repetitions is used to image at least part of the object (2), wherein additional object information, which relates to the position and/or the orientation and/or the feature or at least one further feature of the object (2), is determined by processing this measurement data, wherein the sectional-surface parameter is determined on the basis of said additional object information.

7. Method according to claim 6, **characterised in that** the additional object information specifies a point of intersection of the object with the sectional surface (5) .

8. Method according to claim 7, **characterised in that** during at least one of the repetitions, prior to determining the sectional-surface parameter, the magnetic resonance imaging facility is controlled to acquire auxiliary measurement data relating to an auxiliary sectional surface (6), wherein the auxiliary sectional surface (6) is spaced apart from that sectional surface (5) from which measurement data is acquired in the earlier repetition and/or lies at a defined angle to said surface, whereby an auxiliary point of intersection (7) of the object (2) with the auxiliary sectional surface (6) is determined, whereby the sectional-surface parameter is determined such that the sectional surface comprises the point of intersection and the auxiliary point of intersection (7), or makes a defined angle with a connecting line between the point of intersection and the auxiliary point of intersection (7).

9. Method according to one of the preceding claims, **characterised in that** the steps, or a group of steps that comprises at least determining the relevant sectional-surface parameter and controlling the magnetic resonance imaging facility to acquire the relevant measurement data relating to the corresponding sectional surface (5), are repeated if it is ascertained after acquiring the measurement data that the measurement data does not image the object (2), wherein the sectional-surface parameter is varied according to a preset model or a model defined on the basis of the object information.

10. Method according to one of the preceding claims, **characterised in that** if the measurement data images at least part of the object (2), the measurement data is processed to determine imaging information that specifies what portion of the object (2) is imaged by the measurement data, whereby, in the event that a repetition condition that evaluates the imaging information is satisfied, the steps, or a group of the steps that comprises at least determining the relevant sectional-surface parameter and controlling the magnetic resonance imaging facility to acquire the relevant measurement data relating to the corresponding sectional surface (5), are repeated, wherein the sectional-surface parameter is varied according to a preset model or a model defined on the basis of the object information and/or the measurement data, and in the event that the repetition condition is not satisfied, the two-dimensional image data is calculated on the basis of the previously acquired measurement data.

11. Method according to claim 10, **characterised in that** the imaging information is determined on the basis of at least one feature of the object (2), which is specified by, or determined from, the object information and/or a prior knowledge of the object (2).

12. Method according to claim 10 or 11, **characterised in that** a measure of what portion of the object (2) is imaged by the measurement data is determined as the imaging information, wherein the repetition condition compares the imaging information with a defined limit value or evaluates a convergence criterion, which takes into account a plurality of items of imaging information determined in successive repetitions of the group of steps.

13. Method according to one of the preceding claims, **characterised in that** a destination point (8) is defined in the acquisition volume, wherein the sectional-surface parameter is determined such that the sectional surface comprises the destination point (8).

14. Method according to one of the preceding claims, **characterised in that** the sectional surface (5) is a sectional plane, wherein the sectional surface (5) lies parallel to a central ray (16) of an X-ray source (10) of the X-ray facility.

15. Method according to one of the preceding claims, **characterised in that** an object acquisition facility can be used to determine position information that specifies the position of a marker element or position acquisition element arranged on the object (2) in the acquisition volume, wherein the sectional-surface parameter is determined on the basis of the position information.

16. Method according to one of the preceding claims, **characterised in that** two of the X-ray images (1) are acquired at different acquisition angles, whereby a three-dimensional position in the acquisition volume is determined for at least one defined region of the object (2) from the X-ray images (1) as the object information, whereby the sectional-surface parameter is determined on the basis of the three-dimensional position.

17. Method according to claim 16, **characterised in that** an X-ray facility is used that comprises a plurality of pairs of X-ray sources (10) and X-ray detectors (11), the acquisition angles of which differ from one another, wherein the X-ray images (1) acquired at the different acquisition angles are acquired by different pairs.

18. Method according to claim 16, **characterised in that** the X-ray images (1) acquired at the different acquisition angles are captured by an X-ray facility that is mounted such that it can rotate and/or be displaced relative to the acquisition volume.

19. Method according to one of the preceding claims, **characterised in that** a curved sectional surface is used.

20. Method according to one of the preceding claims, **characterised in that** an X-ray facility is used that comprises an X-ray detector (11) and an X-ray source (10) whose relative position and/or orientation can be varied with respect to the acquisition volume, wherein in the event of a user action to change the sectional surface acquired by the magnetic resonance imaging facility and/or in the event of a change to the sectional-surface parameter, the position and/or the orientation of the X-ray detector (11) and/or of the X-ray source (10) is automatically adjusted.

21. Combined magnetic resonance imaging and X-ray apparatus that comprises a magnetic resonance imaging facility, an X-ray facility and a control facility (12), wherein the coordinate systems of said X-ray facility and said magnetic resonance imaging facility, which coordinate systems are used for imaging, are registered to each other by a mechanical coupling of the X-ray facility to the magnetic resonance imaging facility, wherein the control facility (12) is configured to implement the method according to one of the preceding claims.

22. Computer program, which can be loaded directly into a memory of a control facility (12) of a combined magnetic resonance imaging and X-ray apparatus (9), which computer program comprises program means in order to implement the steps of the method according to one of claims 1 to 20 when the computer program is executed in the control facility (12) of the combined magnetic resonance imaging and X-ray apparatus (13).

23. Electronically readable data storage medium comprising electronically readable control information stored thereon, which information comprises at least one computer program according to claim 22 and is designed such that when the data storage medium is used in a control facility (12) of a combined magnetic resonance imaging and X-ray apparatus (9), performs a method according to one of claims 1 to 20.

## Revendications

1. Procédé de détermination de données d'image en deux dimensions d'au moins une tranche (5) d'un volume d'acquisition, dans le cadre d'une imagerie à résonnance magnétique par un système (9) combiné à résonnance magnétique et à rayons X, qui comprend un dispositif à résonnance magnétique et un dispositif à rayons X, dont les systèmes de coordonnées utilisés pour l'imagerie sont définis par un accouplement mécanique du dispositif à rayons X au dispositif à résonnance magnétique l'un par rapport à l'autre, comprenant les stades effectués par un dispositif (12) de commande :
- commande du dispositif à rayons X pour l'acquisition d'au moins une image (1) de rayons X, qui reproduit au moins en partie un objet (2), notamment un instrument médical,
- détermination d'au moins une information sur l'objet, qui concerne une position et/ou une orientation et/ou au moins une caractéristique de l'objet (2), par un traitement d'image de l'image (2) à rayons X,
- détermination d'au moins un paramètre de tranche, qui prescrit une disposition de la tranche (5) dans le volume d'acquisition, en fonction de l'information sur l'objet,
- commande du dispositif à résonnance magnétique pour l'acquisition de données de mesure concernant la tranche (5), et
- calcul des données d'image en deux dimensions à partir des données de mesure,
dans lequel la commande du dispositif à résonnance magnétique et/ou le calcul des données d'image en deux dimensions s'effectue en fonction du paramètre de la tranche.

2. Procédé suivant la revendication 1, **caractérisé en ce que** l'on détermine comme information sur l'objet une position (3) ponctuelle respective d'au moins un point de mesure dans l'image (1) de rayons X, qui est dans une position donnée à l'avance par rapport à la reproduction au moins partielle de l'objet (2), après quoi on détermine le paramètre de tranche en fonction de la position (3) du point ou des positions (3) de point de manière à ce que la tranche (5) comprenne au moins un segment d'une ligne de liaison respective entre un élément de détecteur de rayons X, associé au point de mesure respectif, et une source (10) de rayons X du dispositif à rayons X.

3. Procédé suivant la revendication 1 ou 2, **caractérisé en ce que** l'on détermine comme information sur l'objet au moins un vecteur (4) directionnel dans l'image (1) de rayons X, qui décrit une direction donnée à l'avance par rapport à la au moins une reproduction partielle de l'objet (2), après quoi on détermine le paramètre de tranche en fonction du vecteur (4) directionnel ou des vecteurs (4) directionnels de manière à ce qu'au moins une partie respective de la tranche (5) dans l'espace d'acquisition soit parallèle au vecteur directionnel respectif ou fasse un angle donné à l'avance avec celui-ci.

4. Procédé suivant la revendication 2 ou la revendication 3, dans la mesure où elle dépend de la revendication 2, **caractérisé en ce que** l'objet (2) est un cathéter, dans lequel on détermine la position d'une pointe du cathéter dans l'image (1) de rayons X et le au moins un point de mesure se trouve à la position de la pointe du cathéter ou en une autre position donnée à l'avance par rapport à la position de la pointe du cathéter et/ou dans lequel le vecteur (4) directionnel est dirigé suivant une direction longitudinale du cathéter, que l'on détermine dans l'image (1) à rayons X.

5. Procédé suivant l'une des revendications précédentes, **caractérisé en ce que** l'on effectue de manière répétée successivement dans le temps un groupe de stades, qui comprend au moins la détermination du paramètre de tranche respectif et la commande du dispositif à résonnance magnétique pour l'acquisition des données de mesure concernant la tranche (5) respective, dans lequel dans au moins l'une des répétitions, on détermine le paramètre de tranche respectif en fonction des données de mesure acquises dans une répétition antérieure.

6. Procédé suivant la revendication 5, **caractérisé en ce que** l'on reproduit au moins en partie l'objet (2) par les données de mesure acquises dans la répétition antérieure, dans lequel par un traitement de ces données de mesure, on détermine une autre information sur l'objet, qui concerne la position et/ou l'orientation et/ou la au moins une autre caractéristique de l'objet (2), dans lequel on détermine le paramètre de tranche en fonction de l'autre information sur l'objet.

7. Procédé suivant la revendication 6, **caractérisé en ce que** l'autre information sur l'objet décrit un point d'intersection de l'objet avec la tranche (5).

8. Procédé suivant la revendication 7, **caractérisé en ce que**, pendant au moins l'une des répétitions, on commande avant la détermination du paramètre de tranche le dispositif à résonnance magnétique pour l'acquisition de données de mesure auxiliaire concernant une tranche (6) auxiliaire, dans lequel la tranche (6) auxiliaire est à distance de toute tranche (5) dont on a acquise les données de mesure dans la répétition antérieure et/ou fait un angle donné à avance avec celle-ci, après quoi, on détermine un point (7) de coupe auxiliaire de l'objet (2) avec la tranche (6), après quoi on détermine le paramètre de tranche de manière à ce que la tranche comprenne le point de coupe et le point (7) de coupe auxiliaire ou fasse un angle donné à l'avance avec une ligne de liaison entre le point de coupe et le point (7) de coupe auxiliaire.

9. Procédé suivant l'une des revendications précédentes, **caractérisé en ce que** l'on répète le ou un groupe de stades, qui comprend au moins la détermination du paramètre de tranche respectif et la commande du dispositif à résonnance magnétique pour l'acquisition des données de mesure, concernant la tranche (5) respective si, après l'acquisition des données de mesure, on détermine que les données de mesure ne reproduisent pas l'objet (2), dans lequel on fait varier le paramètre de tranche suivant un modèle donné à l'avance de manière fixe ou en fonction de l'information sur l'objet.

10. Procédé suivant l'une des revendications précédentes, **caractérisé en ce que**, si les données de mesure reproduisent au moins en partie l'objet (2), on détermine par un traitement des données de mesure une information de reproduction, qui décrit la proportion de l'objet (2) reproduite par les données de mesure, après quoi, si une condition de répétition exploitant l'information de reproduction est satisfaite, on répète le ou un groupe des stades qui comprend au moins la détermination du paramètre de tranche respectif et la commande du dispositif à résonnance magnétique pour l'acquisition des données de mesure concernant la tranche (5) respective, dans lequel on fait varier le paramètre de tranche suivant un modèle donné à l'avance de manière fixe ou en fonction de l'information sur l'objet et/ou des données de mesure et, si la condition de répétition n'est pas satisfaite, on calcule les données d'image en deux dimensions en fonction des données de mesure acquises précédemment.

11. Procédé suivant la revendication 10, **caractérisé en ce que** l'on détermine l'information de reproduction en fonction au moins une caractéristique de l'objet (2), qui est décrite par l'information sur l'objet et/ou par un savoir antérieur sur l'objet ou qui s'en détermine.

12. Procédé suivant la revendication 10 ou 11, **caractérisé en ce que** l'on détermine comme information de reproduction une mesure, qui reproduit la proportion de l'objet (2) reproduite par les données de mesure, dans lequel la condition de répétition compare l'information de reproduction à une valeur limite donnée à l'avance ou exploite un critère de convergence, qui prend en compte plusieurs informations de reproduction, déterminées dans des répétitions successives du groupe des stades.

13. Procédé suivant l'une des revendications précédentes, **caractérisé en ce que** l'on prescrit un point (8) cible dans le volume d'acquisition, dans lequel on détermine le paramètre de tranche de manière à ce que la tranche comprenne le point (8) cible.

14. Procédé suivant l'une des revendications précédentes, **caractérisé en ce que** la tranche (5) est un plan de coupe, la tranche (5) étant parallèle à un rayon (16) central de la source (10) de rayons X du dispositif à rayons X.

15. Procédé suivant l'une des revendications précédentes, **caractérisé en ce que** par un dispositif de détection d'objet, on détermine une information de position, qui décrit la position d'un élément de repérage disposé sur l'objet (2) ou d'un élément de détection de position dans le volume d'acquisition, dans lequel on détermine le paramètre de tranche en fonction de l'information de position.

16. Procédé suivant l'une des revendications précédentes, **caractérisé en ce que** l'on acquiert deux des images (1) de rayons X sous des angles d'enregistrement différents, après quoi on détermine comme information sur l'objet une position en trois dimensions dans le volume d'acquisition pour au moins une partie donnée à l'avance de l'objet (7) à partir des images (1) de rayons X, après quoi on détermine le paramètre de tranche en fonction de la position en trois dimensions.

17. Procédé suivant la revendication 16, **caractérisé en ce que** l'on utilise un dispositif à rayons X ayant plusieurs paires de sources (10) de rayons X et de détecteurs, dont les angles d'enregistrement sont différents les uns des autres, dans lequel on acquiert par des paires différentes les images (1) de rayons X acquises sous des angles d'enregistrement différents.

18. Procédé suivant la revendication 16, **caractérisé en ce qu'**on acquiert les images (1) de rayons X acquises sous des angles d'enregistrement différents par un dispositif à rayons X, qui est monté tournant et/ou coulissant par rapport au volume d'acquisition.

19. Procédé suivant l'une des revendications précédentes, **caractérisé en ce que** l'on utilise une tranche incurvée.

20. Procédé suivant l'une des revendications précédentes, **caractérisé en ce que** l'on utilise un dispositif à rayons X, qui a un détecteur (11) de rayons X et une source (10) de rayons X, dont la position relative et/ou l'orientation par rapport au volume d'acquisition peuvent être modifiées, dans lequel, lors d'une manipulation par un utilisateur pour modifier la tranche prise par le dispositif à résonnance magnétique et/ou lors d'une modification du paramètre de tranche, on adapte automatiquement la position et/ou l'orientation du détecteur (11) de rayons X et/ou de la source (10) de rayons X.

21. Installation combinée à résonnance magnétique et à rayons X, comprenant un dispositif à résonnance magnétique, un dispositif à rayons X et un dispositif (12) de commande, dans laquelle les systèmes de coordonnées utilisés pour l'imagerie du dispositif à rayons X et du dispositif à résonnance magnétique sont définis par un accouplement mécanique du dispositif à rayons X au dispositif à résonnance magnétique l'un à l'autre, dans laquelle le dispositif (12) de commande est configuré pour effectuer le procédé suivant l'une des revendications précédentes.

22. Programme d'ordinateur, qui peut être chargé directement dans une mémoire d'un dispositif (12) de commande d'une installation (9) combinée à résonnance magnétique et à rayons X, comprenant des moyens de programme pour effectuer les stades du procédé suivant l'une des revendications 1 à 20, lorsque le programme d'ordinateur est réalisé dans le dispositif (12) de commande de l'installation (13) combinée à résonnance magnétique et à rayons X.

23. Support de données déchiffrable électroniquement, sur lequel sont mises en mémoire des informations de commande déchiffrable électroniquement, qui comprennent au moins un programme d'ordinateur suivant la revendication 22 et qui sont conformées de manière à ce qu'elles effectuent, lors de l'utilisation du support de données dans un dispositif (12) de commande d'une installation (9) combinée à résonnance magnétique et à rayons X, le procédé suivant l'une des revendications 1 à 20.
